# EUROPEAN PATENT APPLICATION

(11) **EP 4 253 418 A1**
(43) Date of publication of application: **04.10.2023**
(21) Application number: 22382294.1
(22) Date of filing: 29.03.2022
(51) Int. Cl.: C07K 16/28, C07K 16/32, A61K 35/17, A61K 39/00, A61K 39/395, A61P 35/00

(54) **IMMUNE CELLS EXPRESSING CHIMERIC ANTIGEN RECEPTORS AND BISPECIFIC ANTIBODIES AND USES THEREOF**

(71) Applicant: Fundació Privada Institut d'Investigació Oncològica de Vall-Hebron, 08035 Barcelona (ES); Fundació Institut Mar d'Investigacions Mèdiques (IMIM), 08003 Barcelona (ES); Institució Catalana de Recerca i Estudis Avançats, 08010 Barcelona (ES)
(72) Inventor: ARRIBAS LÓPEZ, Joaquín, E-08035 Barcelona (ES); ROMÁN ALONSO, Macarena, E-08035 Barcelona (ES); GRINYÓ I ESCUER, Ariadna, E-08035 Barcelona (ES); DURO SÁNCHEZ, Santiago, E-08003 Barcelona (ES); RIUS RUIZ, Irene, E-08035 Barcelona (ES); NOGALES TRALLERO, Vanesa, E-08003 Barcelona (ES)
(74) Representative: ABG Intellectual Property Law, S.L.

(57) **Abstract**

The present invention relates to immune cells expressing chimeric antigen receptors against p95HER2 and bispecific antibodies for HER2 and CD3 and uses thereof in the treatment of cancer, in particular cancers which overexpress p95HER2.

## Description

### FIELD OF THE INVENTION

The present invention is comprised within the field of biotechnology and biomedicine. It specifically relates to immune cells expressing specific chimeric antigen receptors against the p95 fragment of HER2 and bispecific antibodies for HER2 and CD3 and uses thereof in in the treatment of cancer.

### BACKGROUND ART

Redirection of immune cells against tumor-associated or tumor-specific antigens is a successful therapeutic strategy against certain hematologic malignancies. In contrast, it has failed against solid tumors so far. T cells can be redirected using bispecific antibodies, commonly known as bispecific T cell engagers (BiTEs) or T cell bispecific antibodies (TCBs), which simultaneously bind a tumor antigen and an invariant subunit of the T cell receptor (TCR), typically CD3. Alternatively, T cells can be manipulated to express chimeric antigen receptors (CARs), engineered proteins that contain the antigen-binding domain of an antibody and signaling domains of the TCR and co-activator receptors.

In contrast with the successes on hematologic malignancies, so far, T cell redirection has failed against solid tumors. The causes of this failure include down-modulation of the antigen against which T cells are redirected, heterogeneity of the expression of said antigen, and the immunosuppressive environment that tumors establish.

While improvement in the design of BiTEs to overcome such evasion mechanisms has inherent limitations, CAR T cells can be used as platforms to deliver additional antitumor factors such as cytokines or antibodies. The cytokines produced by these so-called fourth generation CARs include IL-12 and IL-15. The antibodies delivered by fourth generation CARs T cells include blocking antibodies targeting immune checkpoint inhibitors (and bispecific antibodies targeting tumor antigens (Choi et al., 2019, Nature Publishing Group 37, 1049-1058). Armored CARs constitute an expandable platform to counteract the aforementioned mechanisms of resistance.

Given the scarcity of tumor-specific antigens, the vast majority of CARs developed to date have been directed against tumor-associated antigen. As a consequence, frequent and serious side effects caused by on-target off-tumor activity have limited their use. Although, in principle, they should be devoid of side effects, CARs directed against the few tumor-specific antigens available have not been effective yet. This failure is, at least in part, due to the low levels and/or heterogeneous expression of these tumor-specific antigens. Tumor cells eventually escape by down-modulating them, or non-expressing tumor cells are rapidly selected. To take advantage of the specificity of tumor-specific antigens and combine it with the potency of targeting tumor-associated antigens, fourth generation CAR T cells expressing BiTEs have been developed, using as a proof-of-concept glioblastoma.

The gene encoding the tyrosine kinase receptor HER2 is amplified in approximately 3% of all tumors. In some tumors, such as those affecting the breast and the upper gastrointestinal tract, the frequency of HER2 amplification reaches ~15%. Approximately one third of these tumors also express a truncated form of HER2 known as p95HER2. While HER2 is also expressed in normal epithelium, albeit at much lower levels than in HER2-amplified tumors, p95HER2 is a bona fide tumor-specific antigen. Consistently, we have previously shown that a specific TCB effectively redirects lymphocytes against p95HER2-positive breast tumors but does not target cells expressing normal levels of HER2

Several second-generation CARs targeting p95HER2 have been generated. These CARs have been shown to be very effective against cell lines *in vitro* and *in vivo,* but with partial effect against patient-derived tumor xenografts, indicating that their efficacy in the clinic will be limited. Therefore, given the limitations of the present cell therapies, either CAR or BiTEs, alternatives are required which take advantage of the specificity of p95HER2 targeting and the wider effect of HER2 targeting.

### SUMMARY OF THE INVENTION

The present invention discloses armored CAR immune cells that target p95HER2 which, in order to potentiate their therapeutic antitumoral effect, have been further modified so that are capable of secreting a bispecific T cell engager (BiTE) that recruits immune cells to target HER2. It is expected that this p95HER2 CARs secreting HER2 BiTEs will combine the specificity, and therefore the safety, of targeting p95HER2 with the potency of targeting HER2 locally.

In other words, the present invention takes advantage of a tumor-specific antigen (p95HER2) heterogeneously expressed in tumors, to safely trigger the local targeting of a more abundant antigen, but which is also expressed in normal tissues (HER2). The invention significantly increases the antitumor effect of previously described second generation p95HER2 CAR immune cells, and will presumably avoid the off-tumor side effects typical from HER2 targeted therapies.

Therefore, a first aspect of the present invention refers to an immune cell which expresses
(i) a chimeric antigen receptor comprising:
   ∘ an antigen binding domain specific for p95HER2,
   ∘ a transmembrane domain and
   ∘ at least one intracellular signaling domain and/or a costimulatory domain
      and
(ii) a bispecific antibody comprising:
   ∘ a first antigen-binding region which specifically binds to HER2 and
   ∘ a second antigen-biding region which specifically binds CD3.

A further aspect of the present invention relates to a nucleic acid construct comprising
(i) a first region encoding a chimeric antigen receptor, said chimeric antigen receptor comprising:
   ∘ an antigen binding domain specific for p95HER2,
   ∘ a transmembrane domain and
   ∘ at least one intracellular signaling domain and/or a costimulatory domain
      and
(ii) a second region encoding a bispecific antibody, said bispecific antibody comprising:
   ∘ a first antigen-binding region which specifically binds to HER2 and
   ∘ a second antigen-biding region which specifically binds CD3.
      or a nucleic acid composition comprising
      (i) a first polynucleotide encoding a chimeric antigen receptor, said chimeric antigen receptor comprising:
         ∘ an antigen binding domain specific for p95HER2,
         ∘ a transmembrane domain and
         ∘ at least one intracellular signaling domain and/or a costimulatory domain
            and
      (ii) a second polynucleotide encoding a bispecific antibody, said bispecific antibody comprising:
         ∘ a first antigen-binding region which specifically binds to HER2 and
         ∘ a second antigen-biding region which specifically binds CD3.

Another aspect of the present invention relates to a vector containing the nucleic acid construct according to the invention or a vector composition comprising vectors in which the first and second nucleic acids of the composition are found in different vectors.

Yet another aspect of the present invention relates to a polypeptide encoded by the nucleic acid construct according to the invention.

One more aspect relates to a method for obtaining a cell expressing
(i) a chimeric antigen receptor comprising:
   ∘ an antigen binding domain specific for p95HER2,
   ∘ a transmembrane domain and
   ∘ at least one intracellular signaling domain and/or a costimulatory domain
      and
(ii) a bispecific antibody comprising:
   ∘ a first antigen-binding region which specifically binds to HER2 and
   ∘ a second antigen-biding region which specifically binds CD3.
      comprising inserting into the cell a nucleic acid construct or a nucleic acid composition according to the invention or a vector or vector composition according to the invention.

### BRIEF DESCRIPTION OF THE FIGURES

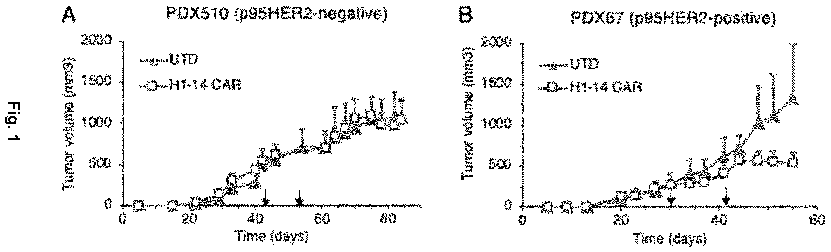

### Figure 1 - Effect of p95HER2 CAR Ts on PDXs.

Two patient-derived tumor xenografts (PDTXs), negative **(A)** and positive **(B)** for p95HER2, were orthotopically injected into NSG mice. At the time points indicated by the arrows, 3 10⁶ T cells expressing the H1-14 p95HER2 CAR were injected intravenously. Tumor volumes are represented as averages ± SD (standard deviation) (n=6 per arm). UTD, control non-targeting CAR.

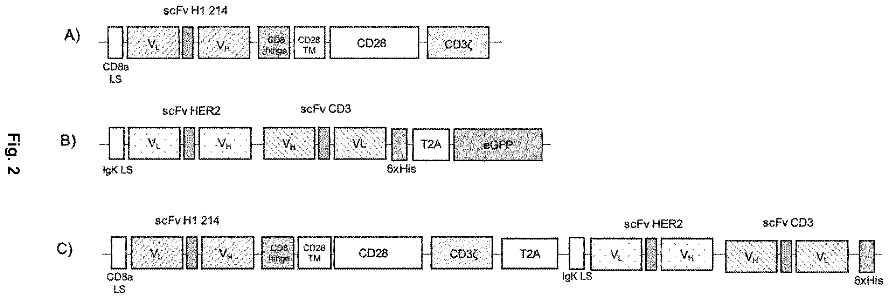

### Figure 2 - Schematic representation of the three constructs described in the present document.

(A) H1-14 p95HER2 CAR or "CAR", **(B)** HER2-CD3 BiTe or "BiTe" and **(C)** p95HER2.CAR - HER2-CD3.BiTE or "CAR-BiTe". Abbreviations used in the diagrams: ScFv = single chain Fragment Variable, VL = light chain, VH = heavy chain, LS = leader sequence, TM = transmembrane domain, 6xHis = Peptide Tag consisting in 6 Histidine repeats, T2A = self-cleaving peptide.

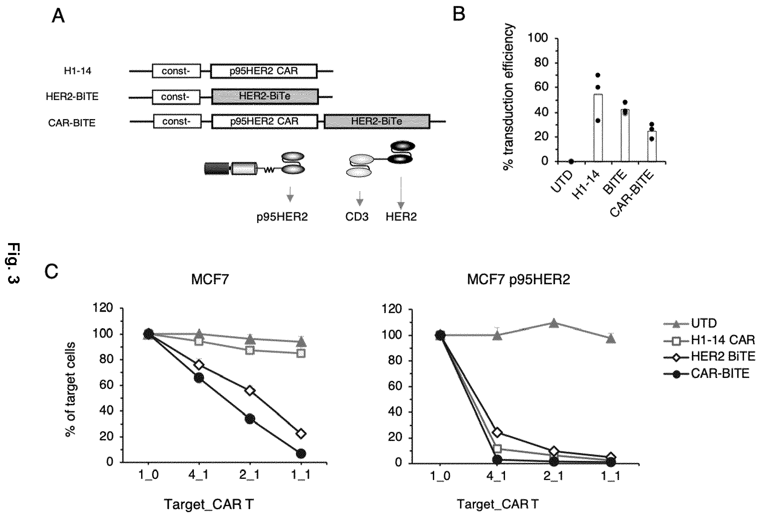

### Figure 3 - In vitro characterization of p95HER2.CAR - HER2-CD3.BiTe Immune cells (CAR-BITE)

**(A)** Simplified schemes of the indicated retroviral vectors and **(B)** their transduction efficiencies on T cells at day 4 post-transduction; percentage of positive-CAR or percentage of positive-EGFP from total T cells are indicated. **(C)** MCF7 cells expressing empty vector or the same vector expressing p95HER2 (Parra-Palau et al., 2014) were co-cultured with different ratios of p95HER2 CAR T, HER2 BiTe secreting T or p95HER2.CAR- HER2.BiTe T cells for 48 h. Then, viable target cells were quantified by flow cytometry using EpCAM as an epithelial cell marker. Results are expressed as averages of three biological replicas. Const = constitutive promoter.

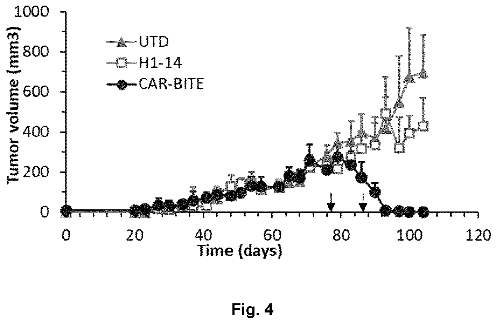

### Figure 4 - In vivo effect of p95HER2.CAR-HER2.BiTe on PDXs.

Fragments of a PDTX with heterogeneous expression of p95HER2, were orthotopically injected into NSG mice. At the time points indicated by the arrows, 3×10⁶ T cells expressing the H1-14 p95HER2 CAR (H1-14) or p95HER2.CAR-HER2.BiTe (CAR-BiTE) were injected intravenously. Tumor volumes are represented as averages ± SD (standard deviation) (n=6 per arm). UTD, control non-targeting CAR.

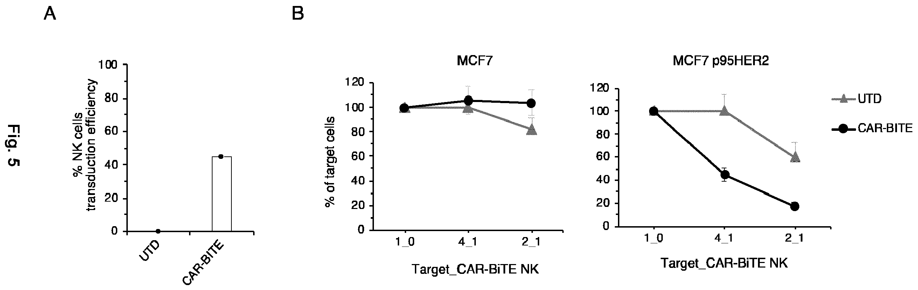

### Figure 5 - In vitro characterization of p95HER2.CAR - HER2-CD3.BiTe NK cells (CAR-BITE NKs).

**(A)** Transduction efficiencies of NK cells at day 4 post-transduction with the retroviral vectors indicated in Figure 3A; percentage of positive-CAR from total NK cells are indicated. **(B)** MCF7 cells expressing empty vector or the same vector expressing p95HER2 (Parra-Palau et al., 2014) were co-cultured with different ratios of p95HER2 CAR - HER2 BiTe secreting NK cells or untransduced NK cells for 24 h. Then, viable target cells were quantified by flow cytometry using EpCAM as an epithelial cell marker. Results are expressed as averages of three technical replicas.

### DETAILED DESCRIPTION OF THE INVENTION

Given the limitations of the current cell therapies for the treatment of tumors, the present invention relates to fourth generation CAR immune cells expressing bispecific T cell engagers (BiTEs), in order to take advantage of the specificity of tumor-specific antigens and combine it with the potency of targeting tumor-associated antigens homogeneously expressed at higher levels in tumor cells. The present work discloses immune cells expressing a chimeric antigen receptor specific for p95HER2 and a bispecific antibody targeting HER2 and CD3.

Unless otherwise defined, all technical terms used herein have the same meaning as commonly understood by one of ordinary skill in the art to which this invention belongs.

All the embodiments and definitions disclosed in the context of one aspect of the invention are also applicable to the other aspects of the invention.

### Immune cell of the invention

Therefore, a first aspect of the present invention relates to an immune cell, from here onwards the immune cell of the invention, which expresses
(i) a chimeric antigen receptor (CAR) comprising:
   ∘ an antigen binding domain specific for p95HER2,
   ∘ a transmembrane domain and
   ∘ at least one intracellular signaling domain and/or a costimulatory domain
      and
(ii) a bispecific antibody comprising:
   ∘ a first antigen-binding region which specifically binds to HER2 and
   ∘ a second antigen-biding region which specifically binds CD3.

As used herein, "immune cell" refers to a cell that plays a role in the immune response. immune cells are of hematopoietic origin, and include lymphocytes, such as B cells and T cells; natural killer cells; myeloid cells, such as monocytes, macrophages, eosinophils, mast cells, basophils, and granulocytes. In some embodiments, the cell is a T cell; a NK cell; a NKT cell; lymphocytes, such as B cells and T cells; and myeloid cells, such as monocytes, macrophages, eosinophils, mast cells, basophils, and granulocytes.

In a particular embodiment of the method of the invention the immune cell is a T cell or a natural killer (NK) cell.

As used herein, the term "T cell" refers to a type of lymphocyte that matures in the thymus. T cells play an important role in cell-mediated immunity and are distinguished from other lymphocytes such as B lymphocytes by the presence of T cell receptors on the cell surface. T cells can also be isolated or obtained from commercially available sources. T cells are of any type expressing CD3, including helper T cells (CD4+ cells), cytotoxic T cells (CD8+ cells), natural killer T cells, regulatory T cells (Tregs) and gamma-delta T cells. "Cytotoxic cells" include CD8+ T cells, natural-killer (NK) cells, and neutrophils capable of mediating a cytotoxic response. The terms "T cell" and "T lymphocyte" are interchangeable and are used interchangeably herein.

Natural killer cells or "NK cells" are well known in the art. In one embodiment, natural killer cells include cell lines, such as NK- 92 cells. Further examples of NK cell lines include NKG, YT, NK-YS, HANK-1, YTS cells, and NKL cells. NK cells can be detected by specific surface markers, such as CD16, CD56, and CD8 in humans. NK cells do not express T-cell antigen receptors, the pan T marker CD3, or surface immunoglobulin B cell receptors.

Natural killer T (NKT) cells are a heterogeneous group of T cells that share properties of both T cells and natural killer cells. Thus, NKT cells are a subset of T cells that coexpress an αβ T-cell receptor, but also express a variety of molecular markers that are typically associated with NK cells, such as NK1. Many of these cells recognize the non-polymorphic CD1d molecule, an antigen-presenting molecule that binds self and foreign lipids and glycolipids. They constitute only approximately 0.1% of all peripheral blood T cells. Natural killer T cells should not be confused with natural killer cells.

### Immune cell of the invention - Chimeric antigen receptor

As used herein, a "chimeric antigen receptor" or "CAR" also known as chimeric T cell receptors, a T-body, artificial T cell receptors and chimeric immune receptors (CIR), are engineered receptors, which graft an arbitrary specificity onto an immune effector cell. In a classical CAR, the specificity of a monoclonal antibody is grafted on to a T cell. CARs are therefore fusion proteins which comprise at least, an extracellular domain or antigen binding domain capable of binding to an antigen, a transmembrane domain derived from a polypeptide different from a polypeptide from which the extracellular domain is derived, and at least one intracellular costimulatory domain.

According to the present invention, the expressions "extracellular domain", antigen-binding domain", "antigen-binding region", "antigen-binding fragment" or "antibody fragment" are used interchangeably and refer to any oligopeptide or polypeptide that can bind to a certain antigen. In the present invention said antigen may be p95HER2, HER2 or CD3, i.e., the antigen-binding domain or antigen-binding region is specific for p95HER2, HER2 or CD3.

The antigen-binding domain may comprise an antibody fragment, which refers to at least one portion of an intact antibody, or recombinant variants thereof, for example an antigen variable region of an intact antibody that is sufficient to allow recognition and specific binding of an antibody fragment to a target. The antigen-binding domain of the invention comprises at least a VH region and a VL region. Examples of antibody fragments include, but are not limited to Fab, Fab'-, F(ab')2 and Fv fragments, ScFv antibody fragments and linear antibodies. Within the context of the present invention, the antigen-binding domain or antibody fragment comprise at least one VH and one VL regions, but it may comprise two VL regions and two VH regions. Thus, for example, in a particular embodiment, the antigen-binding domain specific for p95HER2 of the CAR expressed by the immune cell of the invention is a ScFv, and therefore, it will comprise only one VL and one VH regions. In another embodiment, the antigen-binding domain specific for p95HER2 of the CAR expressed by the immune cell of the invention is a Fab fragment, in which case it will comprise one VL and VH (Fab or Fab') or two VH and two VL regions (Fab2, or F(ab')2).

In a particular embodiment, the antigen-binding domain is humanized.

As used herein, "humanized" forms of non-human (e.g., murine) antibodies or antigen-binding domains are chimeric antibodies or antigen-binding domains that contain minimal sequence, or no sequence, derived from non-human immunoglobulin. For the most part, humanized antibodies or antigen-binding domains are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies or antigen-binding domains can comprise residues that are not found in the recipient antibody or in the donor antibody. These modifications are generally made to further refine antibody or antigen-binding domain performance. In general, the humanized antibody or antigen-binding domain will comprise substantially all of at least one, and typically two, variable domains, in which all or substantially all of the hypervariable loops correspond to those of a nonhuman immunoglobulin and all or substantially all of the FR residues are those of a human immunoglobulin sequence. The humanized antibody can also comprise at least a portion of an immunoglobulin constant region (Fc), typically that of a human immunoglobulin.

The terms "HER2" and "HER2 receptor" are used interchangeably herein, and refer to the ErbB2 protein (also referred to as HER2/neu in the literature). As used herein, the terms are intended to include variants (e.g., splice variants), isoforms, and homologs of HER2 (both orthologs and paralogs). In some aspects, binding of an anti-HER2 binding molecule disclosed herein to HER2 inhibits the growth of cells expressing HER2 (i.e. typically tumor cells, and in particular cancer cells expressing high levels of HER2) by inhibiting formation of heteromeric complexes between HER2 and other ErbB family members, e.g. inhibiting heterodimerization with EGFR or HER3.

HER2 is a receptor tyrosine kinase and is composed of an extracellular domain (ECD), which consists of (i) two leucine-rich domains (domain I/L1 and domain III/L2) responsible for ligand binding, and (ii) two cysteine-rich domains (domain II/CR1 and domain IV/CR2) responsible for receptor dimerization; a transmembrane domain; and an intracellular tyrosine kinase domain. Alternative splice variants of HER2 exist and may also be part of the present invention. HER2 is characterized by a sequence according to the database entry with accession number P04626 of Uniprot, version of 29^{th} September 2021, which is comprised by 1,255 amino acids.

The antigen-binding domain of the CAR expressed by the immune cell of the invention specifically recognizes the amino-terminal fragment of p95HER2, which is located extracellularly. In particular the antigen-binding domain is targeted to the p95HER2 amino acid sequence PIWKFPDE (SEQ ID NO: 5) of the human p95HER2, which is located 34 amino acids from the transmembrane domain which anchors p95HER2 to the membrane.

The term "p95HER2" as used herein refers to a polypeptide that results from the deletion of the N-terminal region of the HER2 receptor protein. The polypeptide is then a carboxy terminal fragment (CTF) of the HER2 receptor protein, which is also known as "611-CTF" or "100-115 kDa p95HER2". p95HER2 is characterized by the amino acid sequence corresponding to amino acids 611-1,255 of HER2, according to SEQ ID NO: 11.
SEQ ID NO: 11

MPIWKFPDEEGACQPCPINCTHSCVDLDDKGCPAEQRASPLTSIISAVVGILLVVVLGVVFGILIKRRQQ
KIRKYTMRRLLQETELVEPLTPSGAMPNQAQMRILKETELRKVKVLGSGAFGTVYKGIWIPDGENVKIPV
AIKVLRENTSPKANKEILDEAYVMAGVGSPYVSRLLGICLTSTVQLVTQLMPYGCLLDHVRENRGRLGSQ
DLLNWCMQIAKGMSYLEDVRLVHRDLAARNVLVKSPNHVKITDFGLARLLDIDETEYHADGGKVPIKWMA
LESILRRRFTHQSDVWSYGVTVWELTFGAKPYDGIPAREIPDLLEKGERLPQPPICTIDVYMIMVCWMID
SECRPRFRELVSEFSRMARDPQRFVVIQNEDLGPASPLDSTFYRSLLEDDDMGDLVDAEEYLVPQQGFFC
PDPAPGAGGMVHHRHRSSSTRSGGGDLTLGLEPSEEEAPRSPLAPSEGAGSDVFDGDLGMGAAKGLQSLP
THDPSPLQRYSEDPTVPLPSETDGYVAPLTCSPQPEYVNQPDVRPQPPSPREGPLPAARPAGATLERPKT
LSPGKNGVVKDFAFGGAVENPEYLTPQGGAAPQPHPPPAFSPAFDNLYYWDQDPPERGAPPSTFKGTPTA
ENPEYLGLDVPV

In a particular embodiment, the antigen-binding domain specific for p95HER2 of the CAR expressed by the immune cell of the invention is a ScFv.

As used herein, a "single chain variable fragment (ScFv)" means a single chain polypeptide derived from an antibody which retains the ability to bind to an antigen. An example of the ScFv includes an antibody polypeptide which is formed by a recombinant DNA technique and in which variable (Fv) regions of immunoglobulin heavy chain (VH chain) and light chain (VL chain) fragments are linked via a spacer sequence. Various methods for preparing a ScFv are known, and include methods described in US Patent No. 4694778, Nature, vol. 334, p. 54454 (1989), and Science, vol. 242, pp. 1038-1041 (1988). In another particular embodiment the antigen-binding domain specific for p95HER2 of the CAR expressed by the immune cell of the invention is a ScFv with the VL region at the N-terminus and the VH region at the C-terminus. In a particular embodiment the antigen-binding domain specific for p95HER2 of the CAR expressed by the immune cell of the invention is a ScFv with the VH region at the N-terminus and the VL region at the C-terminus. In another particular embodiment the antigen-binding domain specific for p95HER2 of the CAR expressed by the immune cell of the invention is a ScFv with the VL region at the N-terminus and the VH region at the C-terminus.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs) or complementary determining regions (CDRs). A single VH or VL domain may be sufficient to confer antigen-binding specificity.

The term "CDRs", "hypervariable region", "HVR", "complementarity determining regions" or as used herein, refers to each of the regions of an antibody variable domain which are hypervariable in sequence and/or form structurally defined loops ("hypervariable loops"). Generally, native four-chain antibodies comprise six CDRs; three in the VH (H1, H2, H3), and three in the VL (L1, L2, L3). Thus, CDRs determine the protein's affinity (roughly, bonding strength) and specificity for specific antigens. The CDRs of the two chains of each pair are aligned by the framework regions, acquiring the function of binding a specific epitope. Consequently, both the heavy variable chain and the light variable chain are characterized by three CDRs, respectively VH-CDR1, VH-CDR2, VH-CDR3 and VL-CDR1, VL-CDR2, VL-CDR3.

As it is used herein, the term "functionally equivalent variant of a CDR sequence" refers to a sequence variant of a particular CDR sequence having substantially similar sequence identity with it and substantially maintaining its capacity to bind to its cognate antigen when being part of an antibody, antibody fragment or antigen-binding domain as the ScFv described herein. For example, a functionally equivalent variant of a CDR sequence may be a polypeptide sequence derivative of said sequence comprising the addition, deletion or substitution of one or more amino acids. In one embodiment, the substitution of one amino acid by other in the functionally equivalent variant is a conservative substitution.

In a particular embodiment the CDR1, CDR2 and CDR3 of the VH region of the ScFv of the CAR expressed by the immune-cell of the invention comprise, respectively, the sequences of SEQ ID NO: 39, 40 and 41 or functionally equivalent variants thereof and/or the CDR1, CDR2 and CDR3 of the VL region of the ScFv of the CAR expressed by the immune cell of the invention comprise respectively, the sequences of SEQ ID NO: 42, 43, and 44 or functionally equivalent variants thereof.

| Region | Sequence | SEQ ID NO: |
|---|---|---|
| VH-CDR1 | TYGMA | SEQ ID NO: 39 |
| VH-CDR2 | TINSNGGKTYHPDSVKG | SEQ ID NO: 40 |
| VH-CDR3 | EGFDY | SEQ ID NO: 41 |
| VL-CDR1 | KASQNVGTAVA | SEQ ID NO: 42 |
| VL-CDR2 | SASNRYT | SEQ ID NO: 43 |
| VL-CDR3 | QQYSTYPLT | SEQ ID NO: 44 |

As used herein, the term "conservative substitution" refers to the replacement of an amino acid by another amino acid having similar chemical properties. Conservative substitution tables providing functionally similar amino acids are well known in the art. The following six groups each contain amino acids that are conservative substitutions for one another:
1) Alanine (A), Serine (S), Threonine (T);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V); and
6) Phenylalanine (F), Tyrosine (Y), Tryptophan (W).

Functionally equivalent variants of a CDR sequence according to the invention include CDR sequences having at least 70% %, at least 75%, at least 80%, at least 85%, at least 90%, at least 91 %, at least 92%, at least 93%, at least 94%, at least 95%, at least 96%, at least 97%, at least 98% or at least 99% sequence identity with the corresponding amino acid sequences shown in one of the above reference sequences. It is also contemplated that functionally equivalent variants of a CDR sequence comprise additions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or at the C-terminus, or both at the N- and C-terminus of the corresponding amino acid sequence shown in one of above referenced sequences. Likewise, it is also contemplated that variants comprise deletions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or at the C-terminus, or both at the N- and C-terminus of the corresponding amino acid sequence shown in one of the above mentioned sequences.

Functionally equivalent variants of a CDR sequence according to the invention will preferably maintain at least 70%, at least 75%, at least 80%, at least 85%, at least 90%, at least 91 %, at least 92%, at least 93%, at least 94%, at least 95%, at least 96%, at least 97%, at least 98%, at least 99%, at least 100%, at least 105%, at least 1 10%, at least 1 15%, at least 120%, at least 125%, at least 130%, at least 135%, at least 140%, at least 145%, at least 150%, at least 200% or more of the capacity of the corresponding amino acid sequence shown in one of SEQ ID NOs: 39-44 to bind to its cognate antigen when being part of an antibody fragment or antigen-binding domain such as the ScFv of the CAR expressed by the immune cell of the invention. This capacity to bind to its cognate antigen may be determined as a value of affinity, avidity, specificity and/or selectivity of the antibody or antibody fragment to its cognate antigen.

The CDR sequences can be determined according to conventional criteria, for example by means of the criteria of IgBLAST: http://www.ncbi.nlm.nih.gov/igblast/ (Ye et al., 2013, Nucleic Acids Res 41 (Web Server issue:W34-40), by following the numbering provided by Kabat et al, Sequences of Proteins of Immunological Interest, 5th ed.,Public Health Service, National Institutes of Health, Bethesda, Md. (1991), or by following the numbering provided by Chothia et al. (1989, Nature 342:877-83).This particular region has been described by Kabat et al., Sequences of Proteins of Immunological Interest, 5th Ed. Public Health Service, National Institutes of Health, Bethesda, Md. (1991) and by Chothia et al., J. Mol. Biol. 196:901-917 (1987), where the definitions include overlapping or subsets of amino acid residues when compared against each other. The exact residue numbers which encompass a particular CDR will vary depending on the sequence and size of the CDR. Those skilled in the art can routinely determine which residues comprise a particular CDR given the variable region amino acid sequence of the antibody. The CDR sequences given herein are generally according to the Kabat definition.

The terms "identity", "identical" or "percent identity" in the context of two or more amino acid or nucleotide sequences, refer to two or more sequences or subsequences that are the same or have a specified percentage of nucleotide or amino acid residues that are the same, when compared and aligned (introducing gaps, if necessary) for maximum correspondence, not considering any conservative substitutions as part of the sequence identity. The percent identity can be measured using sequence comparison software or algorithms or by visual inspection. Various algorithms and software are known in the art that can be used to obtain alignments of amino acid or nucleotide sequences. Publicly available software programs can be used to align sequences. Appropriate parameters for maximal alignment by particular alignment software can be determined by one skilled in the art. In certain embodiments, the default parameters of the alignment software are used. In certain embodiments, the percentage identity "X" of a first nucleotide sequence to a second nucleotide sequence is calculated as 100 x (Y/Z), where Y is the number of nucleotide residues scored as identical matches in the alignment of the first and second sequences (as aligned by visual inspection or a particular sequence alignment program) and Z is the total number of residues in the second sequence. If the second sequence is longer than the first sequence, then the global alignment taken the entirety of both sequences into consideration is used, therefore all letters and null in each sequence must be aligned. In this case, the same formula as above can be used but using as Z value the length of the region wherein the first and second sequence overlaps, said region having a length which is substantially the same as the length of the first sequence.

For instance, 95% identical to a reference sequence according to the present invention, the parameters are set such that the percentage of identity is calculated over the full length of the reference nucleotide sequence and that gaps in homology of up to 5% of the total number of nucleotides in the reference sequence are allowed.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following order in VH (or VL): FR1-H1 (L1)-FR2-H2(L2)-FR3-H3 (L3)-FR4.

As it is used herein, the term "functionally equivalent variant of a FR sequence" refers to a sequence variant of a particular FR sequence having substantially similar sequence identity with it and substantially maintaining its capacity to bind to its cognate antigen when being part of an antibody or antibody-binding domains described herein. For example, a functionally equivalent variant of a FR sequence may be a polypeptide sequence derivative of said sequence comprising the addition, deletion or substitution of one or more amino acids.

In a particular embodiment the FR1, FR2, FR2 and FR4 of the VH region of the CAR expressed by the immune cell of the invention comprise respectively the sequences of SEQ ID NO: 45, 46, 47 and 48 or functionally equivalent variants thereof and/or the FR1, FR2, FR3 and FR4 of the VL region of the CAR expressed by the immune cell of the invention comprise respectively the sequences of SEQ ID NO: 49, 50, 51 and 52 or functionally equivalent variants thereof.

| Region | Sequence | Sequence ID |
|---|---|---|
| VH FR1 | EVQLVESGGGLVQPGGSLRLSCAASGFTFS | SEQ ID NO: 45 |
| VH FR2 | WVRQTPDRRLELVA | SEQ ID NO: 46 |
| VH FR3 | RFTISRDNSKNTLYLQMNSLRAEDTAVYYCTR | SEQ ID NO: 47 |
| VH FR4 | WGQGTLVTVSS | SEQ ID NO: 48 |
| VL FR1 | DIQMTQSPSSLSASVGDRVTITC | SEQ ID NO: 49 |
| VL FR2 | WFQQKPGKAPKILIY | SEQ ID NO: 50 |
| VL FR3 | GVPSRFSGSGSGTDFTLTISSLQPEDFATYYC | SEQ ID NO: 51 |
| VL FR4 | FGQGTKLEIK | SEQ ID NO: 52 |

Functionally equivalent variants of a FR sequence according to the invention include FR sequences having at least approximately 70% , at least 75%, at least 80%, at least 85%, at least 90%, at least 91 %, at least 92%, at least 93%, at least 94%, at least 95%, at least 96%, at least 97%, at least 98% or at least 99% sequence identity with the corresponding amino acid sequences shown in one of the above reference sequences. It is also contemplated that functionally equivalent variants of a FR sequence comprise additions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or at the C-terminus, or both at the N- and C-terminus of the corresponding amino acid sequence shown in one of above referenced sequences. Likewise, it is also contemplated that variants comprise deletions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or at the C-terminus, or both at the N- and C-terminus of the corresponding amino acid sequence shown in one of the above mentioned sequences.

Functionally equivalent variants of a FR sequence according to the invention will preferably maintain at least 70%, at least 75%, at least 80%, at least 85%, at least 90%, at least 91 %, at least 92%, at least 93%, at least 94%, at least 95%, at least 96%, at least 97%, at least 98%, at least 99%, at least 100%, at least 105%, at least 1 10%, at least 1 15%, at least 120%, at least 125%, at least 130%, at least 135%, at least 140%, at least 145%, at least 150%, at least 200% or more of the capacity of the corresponding amino acid sequence shown in one of SEQ ID NOs: 45-52 to bind to its cognate antigen when being part of an antigen-binding domain of the invention. This capacity to bind to its cognate antigen may be determined as a value of affinity, avidity, specificity and/or selectivity of the antibody or antibody fragment to its cognate antigen.

In a particular embodiment the VL of the ScFv of the CAR expressed by the immune cell of the invention comprises sequences having at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity with sequence of SEQ ID NO: 54. In a particular embodiment the VL of the ScFv of the CAR expressed by the immune cell of the invention comprises the sequence of SEQ ID NO: 54. In a particular embodiment the VL of the ScFv of the CAR ScFv of the invention consists or essentially consists of the sequence of SEQ ID NO: 54.

SEQ ID NO: 54
DIQMTQSPSSLSASVGDRVTITCKASQNVGTAVAWFQQKPGKAPKILIYSASNRYTGVPSRFSGSGSGTD
FTLTISSLQPEDFATYYCQQYSTYPLTFGQGTKLEIK

In a particular embodiment the VH of the ScFv of the CAR expressed by the immune cell of the invention comprises sequences having at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity with sequence of SEQ ID NO: 55. In a particular embodiment the VH of the ScFv of the CAR expressed by the immune cell of the invention comprises the sequence of SEQ ID NO: 55. In a particular embodiment the VH of the ScFv of the CAR expressed by the immune cell of the invention consists or essentially consists of the sequence of SEQ ID NO: 55.

SEQ ID NO: 55

EVQLVESGGGLVQPGGSLRLSCAASGFTFSTYGMAWVRQTPDRRLELVATINSNGGKTYHPDSVKGRFTI

SRDNSKNTLYLQMNSLRAEDTAVYYCTREGFDYWGQGTLVTVSS

In a particular embodiment the VL of the ScFv of the CAR expressed by the immune cell of the invention comprises sequences having at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity with the sequence of SEQ ID NO: 54 and the VH of the ScFv of the CAR expressed by the immune cell of the invention comprises sequences having at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity with the sequence of SEQ ID NO: 55.

In a particular embodiment the VL of the ScFv of the CAR expressed by the immune cell of the invention comprises the sequence of SEQ ID NO: 54 and the VH of the ScFv of the CAR expressed by the immune cell of the invention comprises the sequence of SEQ ID NO: 55.

In a particular embodiment the VL of the ScFv of the CAR expressed by the immune cell of the invention consists or essentially consists the sequence of SEQ ID NO: 54 and the VH of the ScFv of the CAR expressed by the immune cell of the invention consists or essentially consists the sequence of SEQ ID NO: 55.

In another particular embodiment of the CAR expressed by the immune cell of the invention the VH and VL regions of the ScFv of the CAR expressed by the immune cell of the invention are connected by a linker region.

The term "flexible polypeptide linker" or "linker" refers to a peptide linker that consists of amino acids such as glycine and/or serine residues used alone or in combination, to link variable heavy and variable light chain regions together; or to link any or the regions of the CAR expressed by the immune cell of the invention.

The linker peptide may have any of a variety of amino acid sequences. Proteins can be joined by a spacer peptide, generally of a flexible nature, although other chemical linkages are not excluded. A linker can be a peptide of between about 6 and about 40 amino acids in length, or between about 6 and about 25 amino acids in length. These linkers can be produced by using synthetic, linker-encoding oligonucleotides to couple the proteins. Peptide linkers with a degree of flexibility can be used. The linking peptides may have virtually any amino acid sequence, bearing in mind that suitable linkers will have a sequence that results in a generally flexible peptide. The use of small amino acids, such as glycine and alanine, are of use in creating a flexible peptide. The creation of such sequences is routine to those of skill in the art.

Suitable linkers can be readily selected and can be of any of a suitable of different lengths, such as from 1 amino acid (e.g., Gly) to 20 amino acids, from 2 amino acids to 15 amino acids, from 3 amino acids to 12 amino acids, including 4 amino acids to 10 amino acids, 5 amino acids to 9 amino acids, 6 amino acids to 8 amino acids, or 7 amino acids to 8 amino acids, and may be 1, 2, 3, 4, 5, 6, or 7 amino acids.

Exemplary flexible linkers include the linker having the sequence TGSTSGSGKPGSGEGS (SEQ ID NO: 53). Suitable linkers include as glycine polymers (G) n, glycine-serine polymers (including, for example, (GS)ₙ, (GSGGS)ₙ (SEQ ID NO: 66) and (GGGS)ₙ (SEQ ID NO: 67), where n is an integer of at least one), glycine-alanine polymers, alanine-serine polymers, and other flexible linkers known in the art. In a particular embodiment the linker comprises a glycine polymer of formula (G₄S)₃. Glycine and glycine-serine polymers are of interest since both of these amino acids are relatively unstructured, and therefore may serve as a neutral tether between components. Glycine polymers are of particular interest since glycine accesses significantly more phi-psi space than even alanine, and is much less restricted than residues with longer side chains. Exemplary flexible linkers include, but are not limited to GGSG (SEQ ID NO: 68), GGSGG (SEQ ID NO: 69), GSGSG (SEQ ID NO: 70), GSGGG (SEQ ID NO: 71), GGGSG (SEQ ID NO: 72), GSSSG (SEQ ID NO: 73), and the like. The ordinarily skilled artisan will recognize that design of a peptide conjugated to any elements described above can include linkers that are all or partially flexible, such that the linker can include a flexible linker as well as one or more portions that confer less flexible structure.

In a particular embodiment, the linker is located between the VH and the VL regions of the ScFv of the CAR expressed by the immune-cell of the invention. In another particular embodiment the VH and VL regions of the ScFv of the CAR expressed by the immune-cell of the invention are connected by a linker region comprising SEQ ID NO: 53. In an embodiment, the ScFv of the CAR expressed by the immune cell of the invention comprises the structure VL-linker-VH. In another embodiment, the ScFv of the CAR expressed by the immune cell of the invention may have the structure VH-linker-VL or VL-linker-VH. In a particular embodiment, the linker is located C-terminally with respect to the VL region and N-terminally with respect to the VH region, that is, VL-linker-VH.

In a particular embodiment the ScFv of the CAR expressed by the immune cell of the invention comprises sequences having at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity with the sequence of SEQ ID NO: 56.
SEQ ID NO: 56

DIQMTQSPSSLSASVGDRVTITCKASQNVGTAVAWFQQKPGKAPKILIYSASNRYTGVPSRFSGSGSGTD
FTLTISSLQPEDFATYYCQQYSTYPLTFGQGTKLEIKTGSTSGSGKPGSGEGSEVQLVESGGGLVQPGGS

LRLSCAASGFTFSTYGMAWVRQTPDRRLELVATINSNGGKTYHPDSVKGRFTISRDNSKNTLYLQMNSLR
AEDTAVYYCTREGFDYWGQGTLVTVSS

In another particular embodiment the ScFv of the CAR expressed by the immune cell of the invention comprises the sequence of SEQ ID NO: 56. In yet another particular embodiment the ScFv of the CAR expressed by the immune cell of the invention consists or consists essentially of the sequence SEQ ID NO: 56.

The second element of the CAR expressed by the immune cell of the invention is a transmembrane domain that is attached to the extracellular domain of the CAR.

As used herein, "transmembrane domain" (TMD) refers to the area of CAR that crosses the cell membrane. The transmembrane domain of the CAR expressed by the immune cell of the invention is the transmembrane domain of a transmembrane protein (e.g., a type I transmembrane protein), an artificial hydrophobic sequence, or a combination thereof. A transmembrane domain can include one or more additional amino acids adjacent to the transmembrane region, e.g., one or more amino acid associated with the extracellular region of the protein from which the transmembrane was derived (e.g., 1, 2, 3, 4, 5, 6, 7, 8, 9, 10 up to 15 amino acids of the extracellular region) and/or one or more additional amino acids associated with the intracellular region of the protein from which the transmembrane protein is derived (e.g., 1, 2, 3, 4, 5, 6, 7, 8, 9, 10 up to 15 amino acids of the intracellular region). In one aspect, the transmembrane domain is one that is associated with one of the other domains of the CAR is used. In some instances, the transmembrane domain can be selected or modified by amino acid substitution to avoid binding of such domains to the transmembrane domains of the same or different surface membrane proteins, e.g., to minimize interactions with other members of the receptor complex. In a particular embodiment, the transmembrane domain of the CAR expressed by the immune cell of the invention is capable of homodimerization with another CAR on the CAR T cell surface. In a different particular embodiment of the CAR expressed by the immune cell of the invention, the amino acid sequence of the transmembrane domain may be modified or substituted so as to minimize interactions with the binding domains of the native binding partner present in the same CAR T.

The transmembrane domain may be derived either from a natural or from a recombinant source. Where the source is natural, the domain may be derived from any membrane-bound or transmembrane protein. In one aspect the transmembrane domain is capable of signaling to the intracellular domain(s) whenever the CAR has bound to a target. Non limiting examples or transmembrane domains of particular use in this invention may include at least the transmembrane region(s) of e.g., the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137, CD154, CD3 zeta, KIRDS2, OX40, CD2, CD27, LFA-1 (CD1 la, CD18), ICOS (CD 278), 4-1BB (CD137), GITR, CD40, CTLA4, BAFFR, HVEM (LIGHTR), SLAMF7, NKp80 (KLRFI), CD160, CD19, IL2R beta, IL2R gamma, IL7Ra, ITGA1, VLA1, CD49a, ITGA4, IA4 CD49D, ITGA6, VLA-6, CD49f, ITGAD, CDGA, CDGA, CD103, ITGAL, CDLa, LFA-1, ITGAM, CDIIb, ITGAX, CDIc, ITGB1, CD29, ITGB2, CD18, LFA-1, LGA ITGB7, TNFR2, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), SLAMF6 (NTB-A) , LyI08), SLAM (SLAMF1, CD150, IPO-3), BLAME (SLAMF8), SELPLG (CD162), LTBR, PAG/Cbp, NKp44, Kp30, NKp46, including NKG2D, and/or a transmembrane domain selected from the transmembrane domain of NKG2C.

In a particular embodiment of the CAR expressed by the immune cell of the invention, the transmembrane domain is selected from the group consisting of the CD4 transmembrane domain, the CD8 transmembrane domain, the CD28 transmembrane domain, the 4-1BB transmembrane domain, the CTLA4 transmembrane domain, the CD27 transmembrane domain and the CD3 zeta transmembrane domain.

In a particular embodiment, the transmembrane domain is the CD28 transmembrane domain. In a particular embodiment, the CD28 transmembrane domain comprises the sequence SEQ ID NO: 57.
SEQ ID NO: 57
FWVLVVVGGVLACYSLLVTVAFIIFWV

In another particular embodiment of the CAR expressed by the immune cell of the invention the transmembrane domain comprises sequences having at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity with the sequence of SEQ ID NO: 57.

In another particular embodiment of the CAR expressed by the immune cell of the invention the transmembrane domain comprises the sequence of SEQ ID NO: 57.

In another particular embodiment of the CAR expressed by the immune cell of the invention the transmembrane domain consists of or essentially consists of the sequence SEQ ID NO: 57.

The CARs according to the present invention comprise at least one intracellular signaling domain and/or costimulatory domain.

"Intracellular signaling domain", as the term is used herein, refers to the intracellular portion of a molecule and more specifically to any oligopeptide or polypeptide known to function as a domain that transmits a signal to cause activation or inhibition of a biological process in a cell. The intracellular signaling domain generates a signal that stimulates the immune effector function of CAR-containing cells, for example, CAR-T cells. The effector function of a T cell, for example, may be cytolytic function or helper activity including the secretion of cytokines. Thus, the intracellular signaling domain may be a portion of a protein which transduces the effector function signal and directs the cell (e.g. T cell) to perform a specialized function.

Generally, the whole intracellular signaling domain can be used; however, it is appreciated that it is not necessary to use the entire domain, provided that whatever part of the signaling domain that is used is still capable of transducing the effector function signal. It will also be appreciated that variants of such intracellular signaling domains with substantially the same or greater functional capability may also be used. By this we include the meaning that the variants should have substantially the same or greater transduction of the effector functional signal. Typically, substantially the same or greater signal transduction includes at least 80%, 85%, 90%, 95%, 100%, 105%, 110%, 115%, or 120%, or more of the signal transduction of the unmodified intracellular signaling domain, wherein signal transduction of the unmodified intracellular signaling domain corresponds to 100%. Methods for assessing transduction of effector function signal are well known to those skilled in the art and include, for example, assessing the amounts and/or activity of molecules (e.g. proteins such as cytokines) that are indicative of the transduced signal. Thus, when the signal is the cytolytic function of a T-cell, the methods may involve measurement of one or more cytokines secreted by the T-cell, which cytokines are known to have a cytolytic activity (e.g. IFN gamma). Another means of assessing the cytolytic function is by CFSE staining and counting positive cells by Flow cytometry or by a chromium release assay as is well known in the art.

Examples of intracellular signaling domains for use in the CAR expressed by the immune cell of the invention include the cytoplasmic sequences of the T cell receptor (TCR) and co-receptors that act in concert to initiate signal transduction following antigen receptor engagement, as well as any derivative or variant of these sequences and any recombinant sequence that has the same functional capability.

It is known that signals generated through the TCR alone are generally insufficient for full activation of a T cell and that a secondary and/or costimulatory signal may also be required. Thus, T cell activation can be said to be mediated by two distinct classes of intracellular signaling sequences: those that initiate antigen-dependent primary activation through the TCR (primary intracellular signaling domains) and those that act in an antigen- independent manner to provide a secondary or costimulatory signal (secondary intracellular signaling domain, such as a costimulatory domain). Costimulatory domains promote activation of effector functions and may also promote persistence of the effector function and/or survival of the cell.

In a particular embodiment of the CAR expressed by the immune cell of the invention, the at least one intracellular signaling domain of the CAR comprises a costimulatory domain, a primary signaling domain, or any combination thereof.

A primary intracellular signaling domain regulates primary activation of the TCR complex either in a stimulatory way, or in an inhibitory way. Primary intracellular signaling domains that act in a stimulatory manner may contain signaling motifs which are known as immunoreceptor tyrosine-based activation motifs or ITAMs (e.g. 2, 3, 4, 5 or more ITAMs). Thus, the intracellular signaling domain may comprise one or more ITAMs. It will be appreciated that one or more ITAMs of the intracellular signaling domain may be modified, for example by mutation. The modification may be used to increase or decrease the signaling function of the ITAM as compared to the native ITAM domain.

Examples of ITAM containing primary intracellular signaling domains that are of particular use in the invention include those of CD3 zeta, Fc receptor gamma, Fc receptor beta, CD3 gamma, CD3 delta, CD3 epsilon, CD5, CD22, CD79a, CD79b, and CD66d. In a particular embodiment the at least one intracellular signaling domain of the CAR expressed by the immune cell of the invention is selected from a group consisting of CD3 zeta, Fc receptor gamma, Fc receptor beta, CD3 gamma, CD3 delta, CD3 epsilon, CD5, CD22, CD79a, CD79b, and CD66d. In a more particular embodiment the at least one intracellular signaling domain of the CAR expressed by the immune cell of the invention is the CD3-zeta intracellular domain.

The term "CD3" refers to the human CD3 protein complex, which is composed of six distinct chains (a CD3y chain (SwissProt P09693), a CD3δ chain (SwissProt P04234), two CD3e chains (SwissProt P07766), and one CD3 zeta chain homodimer (SwissProt P20963) (ε γ: ε δ:ζζ), and which is associated with the T cell receptor α and β chain. The term includes any CD3 variants, isoforms and species homologs which are naturally expressed by cells, including T cells, or are expressed on cells transfected with genes or cDNA encoding the aforementioned chains.

The term "zeta" or alternatively "zeta chain", "CD3-zeta" or "TCR-zeta" is defined as the protein represented by GenBank entry No. BAG36664.1, or equivalent residues from a non-human species, such as a mouse, rodent, monkey, primate, etc., and a "zeta stimulating domain" or alternatively a "CD3 zeta stimulating domain" or "TCR zeta stimulating domain" is defined as amino acid residues of the cytoplasmic domain of the zeta chain that are sufficient for functional transmission of the primary signal required to activate T cells etc. In one aspect, the zeta cytoplasmic domain comprises residues 52 through 164 inclusive of a GenBank entry protein of BAG36664.1, or equivalent residues from a non-human species, for example, a mouse, rodent, monkey, primate, and the like, which are their functional orthologists.

As mentioned above, the intracellular signaling domain may comprise a primary intracellular signaling domain by itself, or it may comprise a primary intracellular signaling domain in combination with one or more secondary intracellular signaling domains, such as one or more costimulatory signaling domains. Thus, the intracellular signaling domain of the CAR may comprise the CD3 zeta signaling domain by itself or in combination with one or more other intracellular signaling domains such as one or more costimulatory signaling domains.

The costimulatory signaling domain refers to a portion of the CAR comprising the intracellular domain of a costimulatory molecule.

The term "co-stimulating molecule" refers to a recognizable T-cell binding partner that specifically binds to a co-stimulating ligand, thereby mediating the co-stimulatory response exerted by the T-cell, such as, but not limited to, proliferation. Co-stimulating molecules are cell surface molecules other than antigen-specific receptors or their ligands, which are necessary for an effective immune response. A costimulatory molecule may be a cell surface molecule other than an antigen receptor or its ligands that is required for an efficient response of immune cells (eg lymphocytes) to an antigen. A costimulatory molecule can be represented in the following protein families: TNF receptor proteins, immunoglobulin-like proteins, cytokine receptors, integrins, lymphocyte activation signaling molecules (SLAM proteins) and NK cell activation receptors. Examples of such molecules include, but are not limited to OX40, ICOS, DAP10, CD27, CD28, CDS, CD30, CD137 (4-1BB), CD40, ICOS, lymphocyte function-associated antigen- 1 (LFA-1), CD2, CD7, LIGHT, NKG2C, GITR, NKG2C, SLAMF7, NKp80, BAFFR, HVEM, BTLA, ICAM-1, LFA-1 (CD11a/CD18), B7- H3, and a ligand that specifically binds with CD83, and the like. For example, CD27 co- stimulation has been demonstrated to enhance expansion, effector function, and survival of human CAR T cells in vitro and augments human T cell persistence and anti-tumour activity in vivo (Song et al. Blood. 2012; 1 19(3):696-706).

In another embodiment, the at least one intracellular signaling domain comprises the intracellular domain of the costimulatory molecules selected from OX40, CD70, CD27, CD28, CD5, ICAM-1, LFA-1 (CD11a/CD18), ICOS (CD278), DAP10, DAP 12, and 4-1BB (CD137), or any combination thereof.

In a particular embodiment, the CAR expressed by the immune cell of the invention comprises the intracellular domain of the costimulatory molecule CD28. In a more particular embodiment, the intracellular domain of a costimulatory molecule comprises the sequence of SEQ ID NO: 58.
SEQ ID NO: 58
RSKRSRLLHSDYMNMTPRRPGPTRKHYQPYAPPRDFAAYRS

In a particular embodiment, the at least one intracellular signaling domain of the CAR expressed by the immune cell of the invention further comprises the CD3-zeta intracellular domain. In one more particular embodiment the at least one intracellular signaling domain of the CAR expressed by the immune cell of the invention further comprises the sequence of SEQ ID NO: 59.
SEQ ID NO: 59

RVKFSRSADAPAYQQGQNQLYNELNLGRREEYDVLDKRRGRDPEMGGKPRRKNPQEGLYNELQKDKMAEA
YSEIGMKGERRRGKGHDGLYQGLSTATKDTYDALHMQALPPR

In one particular embodiment, the at least one intracellular signaling domain of the the CAR expressed by the immune cell of the invention is arranged on an N-terminal side relative to the CD3-zeta intracellular domain.

The intracellular signaling sequences within the intracellular portion of the CAR expressed by the immune cell of the invention may be linked to each other in a random or specified order. Optionally, a short oligo- or polypeptide linker, for example, between 2 and 10 amino acids (e.g., 2, 3, 4, 5, 6, 7, 8, 9, or 10 amino acids) in length may form the linkage between intracellular signaling sequences. In one embodiment, a glycine-serine doublet can be used as a suitable linker. In another embodiment, a single amino acid, such as an alanine or a glycine, can be used as a suitable linker.

In one embodiment, the intracellular signaling domain is designed to comprise two or more, for example 3, 4, 5, or more, costimulatory signaling domains. In an embodiment, the two or more, e.g., 2, 3, 4, 5, or more, costimulatory signaling domains, are separated by a linker molecule, such as one described herein. In one embodiment, the intracellular signaling domain comprises two costimulatory signaling domains. In some embodiments, the linker molecule is a glycine residue. In some embodiments, the linker is an alanine residue.

In preferred embodiments, the intracellular portion of the CAR expressed by the immune cell of the invention comprises:
- the signaling domain of CD3 zeta and the signaling domain of CD28,
- the signaling domain of CD3-zeta and the signaling domain of 4-1 BB,
- the signaling domain of CD3-zeta and the signaling domain of OX40,
- the signaling domain of CD3-zeta and the signaling domain of ICOS,
- the signaling domain of CD3-zeta and the signaling domain of DAP10
- the signaling domain of CD3-zeta, the signaling domain of 4-1 BB and the signaling domain of OX40.
- the signaling domain of 4-1 BB and the signaling domain of CD28.

The intracellular signaling domain may include the entire intracellular portion, or the entire natural intracellular signaling domain, the molecule from which it originates, or a functional fragment thereof.

In another embodiment, the CAR expressed by the immune cell of the invention further comprises a hinge domain between the antigen binding domain and the transmembrane domain.

As used herein, "hinge domain", "hinge region" or "spacer" refers to an amino acid region that allows for separation and flexibility of the binding moiety and the T cell membrane. The length of the flexible hinges also allows for better binding to relatively inaccessible epitopes, e.g., longer hinge domains are allowed for optimal binding. One skilled in the art will be able to determine the appropriate hinge for the given CAR target.

In some cases, the first polypeptide of the CAR expressed by the immune cell of the invention comprises a hinge domain, where the hinge domain is interposed between the antigen-binding domain and the transmembrane domain. In some cases, the hinge domain is an immunoglobulin heavy chain hinge domain. In some cases, the hinge domain is a domain region polypeptide derived from a receptor (e.g., a CD8-derived hinge domain).

The hinge domain can have a length of from about 10 amino acids to about 200 amino acids, preferably, between 50 and 150 amino acids, more preferably between 75 and 125 amino acids.

Exemplary hinge domains include glycine polymers (G) n, glycine-serine polymers (including, for example, (GS)n, (GSGGS)n (SEQ ID NO: 67) and (GGGS)n (SEQ ID NO: 68), where n is an integer of at least one), glycine-alanine polymers, alanine-serine polymers, and other flexible linkers known in the art. Glycine and glycine-serine polymers can be used; both Gly and Ser are relatively unstructured, and therefore can serve as a neutral tether between components. Glycine polymers can be used; glycine accesses significantly more phi-psi space than even alanine, and is much less restricted than residues with longer side chains. Exemplary spacers can comprise amino acid sequences including, but not limited to, GGSG (SEQ ID NO: 69), GGSGG (SEQ ID NO: 70), GSGSG (SEQ ID NO: 701), GSGGG (SEQ ID NO: 712), GGGSG (SEQ ID NO: 73), GSSSG (SEQ ID NO: 734 and the like.

In some cases, the hinge domain in the first polypeptide of the CAR expressed by the immune cell of the invention includes at least one cysteine. For example, in some cases, the hinge domain can include the sequence Cys-Pro-Pro-Cys (SEQ ID NO: 75). If present, a cysteine in the hinge domain of a first CAR can be available to form a disulfide bond with a hinge domain in a second CAR.

Immunoglobulin hinge domain amino acid sequences are known in the art; see, e.g., Tan et al. (1990) Proc. Natl. Acad. Sci. USA 87:162; and Huck et al. (1986) Nucl. Acids Res. 14:1779. As non-limiting examples, an immunoglobulin hinge domain can include one of the following amino acid sequences: DKTHT (SEQ ID NO: 76); CPPC (SEQ ID NO: 75); CPEPKSCDTPPPCPR (SEQ ID NO: 77) (see, e.g., Glaser et al. (2005) J. Biol. Chem. 280:41494); ELKTPLGDTTHT (SEQ ID NO: 77); KSCDKTHTCP (SEQ ID NO: 789); KCCVDCP (SEQ ID NO: 80); KYGPPCP (SEQ ID NO: 81); EPKSCDKTHTCPPCP (SEQ ID NO: 82) (human IgG1 hinge); ERKCCVECPPCP (SEQ ID NO: 83) (human IgG2 hinge); ELKTPLGDTTHTCPRCP (SEQ ID NO: 84) (human IgG3 hinge); SPNMVPHAHHAQ (SEQ ID NO: 85) (human IgG4 hinge); and the like.

The hinge domain can comprise an amino acid sequence of a human IgG1, IgG2, IgG3, or IgG4, hinge domain. The hinge domain can include one or more amino acid substitutions and/or insertions and/or deletions compared to a wild-type (naturally-occurring) hinge domain. For example, His 229 of human IgG1 hinge can be substituted with Tyr, so that the hinge domain comprises the sequence EPKSCDKTYTCPPCP (SEQ ID NO: 86); see, e.g., Yan et al. (2012) J. Biol. Chem. 287:5891).

The hinge domain can comprise an amino acid sequence derived from human CD8 or a variant thereof.

In a particular embodiment, the hinge domain comprises, consists or essentially consists of the CD8 hinge domain.

In another particular embodiment the hinge domain comprises the sequence of SEQ ID NO: 60.
SEQ ID NO: 60
TTTPAPRPPTPAPTIASQPLSLRPEACRPAAGGAVHTRGLDFACD

In a particular embodiment, the hinge domain is the CD8 hinge domain, the transmembrane domain is the CD28 transmembrane domain and the intracellular signaling domain is the CD28 costimulatory domain.

In a particular embodiment, the CAR expressed by the immune cell of the invention comprises the CD8 hinge domain, the CD28 transmembrane domain and the CD3 zeta intracellular signaling domain and the CD28 costimulatory domain.

In another embodiment, the CAR expressed by the immune cell of the invention includes from the N-terminus to the C-terminus an anti-p95HER2 light chain variable domain, a linker domain, an anti- p95HER2 heavy chain variable domain, a CD8 hinge domain, a CD28 transmembrane domain, a CD28 intracellular co-stimulatory signaling domain followed by a CD3 zeta intracellular signaling domain.

In a particular embodiment the CAR expressed by the immune cell of the invention comprises the SEQ ID NO: 61.
SEQ ID NO: 61

DIQMTQSPSSLSASVGDRVTITCKASQNVGTAVAWFQQKPGKAPKILIYSASNRYTGVPSRFSGSGSGTD
FTLTISSLQPEDFATYYCQQYSTYPLTFGQGTKLEIKTGSTSGSGKPGSGEGSEVQLVESGGGLVQPGGS
LRLSCAASGFTFSTYGMAWVRQTPDRRLELVATINSNGGKTYHPDSVKGRFTISRDNSKNTLYLQMNSLR
AEDTAVYYCTREGFDYWGQGTLVTVSSTTTPAPRPPTPAPTIASQPLSLRPEACRPAAGGAVHTRGLDFA
CDFWVLVVVGGVLACYSLLVTVAFIIFWVRSKRSRLLHSDYMNMTPRRPGPTRKHYQPYAPPRDFAAYRS
RVKFSRSADAPAYQQGQNQLYNELNLGRREEYDVLDKRRGRDPEMGGKPRRKNPQEGLYNELQKDKMAEA
YSEIGMKGERRRGKGHDGLYQGLSTATKDTYDALHMQALPPR

### Immune cell of the invention - Bispecific antibody

In addition to the CAR, the immune cell of the invention further expresses a bispecific antibody which binds specifically to HER2 and CD3.

The term "antibody" (Ab) in the context of the present invention refers to an immunoglobulin molecule, a fragment of an immunoglobulin molecule, or a derivative of either thereof, which has the ability to specifically bind to an antigen under typical physiological conditions. The variable regions of the heavy and light chains of the immunoglobulin molecule contain a binding domain that interacts with an antigen. The constant regions of the antibodies (Abs) may mediate the binding of the immunoglobulin to host tissues or factors, including various cells of the immune system. As indicated above, the term antibody herein, unless otherwise stated or clearly contradicted by context, includes fragments of an antibody that are antigen-binding fragments which have been previously defined. It also should be understood that the term antibody, unless specified otherwise, also includes polyclonal antibodies, monoclonal antibodies (mAbs), antibody-like polypeptides, such as chimeric antibodies and humanized antibodies, and antibody fragments retaining the ability to specifically bind to the antigen (antigen-binding fragments) provided by any known technique.

The term "bispecific antibody" refers to, in the context of the present invention, an antibody having two different antigen-binding regions defined by different antibody sequences.

In a particular embodiment of the bispecific antibody expressed by the immune cell of the invention the first and second antigen binding regions of the bispecific antibody are ScFv.

In another particular embodiment of the bispecific antibody expressed by the immune cell of the invention:
- the VH region of the ScFv of the first antigen binding region has CDR1, CDR2 and CDR3 which comprise respectively the sequences of SEQ ID NO:1, SEQ ID NO:2 and SEQ ID NO:3, or functionally equivalents thereof;
- the VL region of the ScFv of the first antigen binding region has CDR1, CDR2 and CDR3 which comprise respectively the sequences of SEQ ID NO:4, SEQ ID NO: 100 and SEQ ID NO:6 or functionally equivalents thereof;
- the VH region of the ScFv of the second antigen binding region has CDR1, CDR2 and CDR3 which comprise respectively the sequences of SEQ ID NO:7, SEQ ID NO:8 and SEQ ID NO:9 or functionally equivalents thereof; and/or
- the VL region of the ScFv of the second antigen binding region has CDR1, CDR2 and CDR3 which comprise respectively the sequences of SEQ ID NO:10, SEQ ID NO: 101 and SEQ ID NO: 12 or functionally equivalent thereof.

| Region | | Sequence | Sequence ID |
|---|---|---|---|
| First Antigen binding region | VH CDR1 | DTYIH | SEQ ID NO: 1 |
| | VH CDR2 | RIYPTNGYTRYADSVKG | SEQ ID NO: 2 |
| | VH CDR3 | WGGDGFYAMDV | SEQ ID NO: 3 |
| | VL CDR1 | RASQDVNTA | SEQ ID NO: 4 |
| | VL CDR2 | SASFLYS | SEQ ID NO: 100 |
| | VL CDR3 | QQHYTTPPT | SEQ ID NO: 6 |
| Second Antigen binding region | VH CDR1 | RYTMH | SEQ ID NO: 7 |
| | VH CDR2 | YINPSRGYTNYNQKFKD | SEQ ID NO: 8 |
| | VH CDR3 | YYDDHYCLDY | SEQ ID NO: 9 |
| | VL CDR1 | RASSSVSYMN | SEQ ID NO: 10 |
| | VL CDR2 | DTSKVAS | SEQ ID NO: 101 |
| | VL CDR3 | QQWSSNPLT | SEQ ID NO: 12 |

In a further particular embodiment of the bispecific antibody expressed by the immune cell of the invention:
- the VH region of the ScFv of the first antigen binding region has FR1, FR2, FR3 and FR4 which comprise respectively the sequences of SEQ ID NO:13, SEQ ID NO:14, SEQ ID NO:15 and SEQ ID NO:16 or functionally equivalents thereof;
- the VL region of the ScFv of the first antigen binding region has FR1, FR2, FR3 and FR4 which comprise respectively the sequences of SEQ ID NO:17, SEQ ID NO:18, SEQ ID NO:19 or SEQ ID NO:20 or functionally equivalents thereof;
- the VH region of the ScFv of the second antigen binding region has FR1, FR2, FR3 and FR4 which comprise respectively the sequences of SEQ ID NO:21, SEQ ID NO:22, SEQ ID NO:23 and SEQ ID NO:24 or functionally equivalents thereof; and/or
- the VL region of the ScFv of the second antigen binding region has FR1, FR2, FR3 and FR4 which comprise respectively the sequences of SEQ ID NO:25, SEQ ID NO:26, SEQ ID NO:27 or SEQ ID NO:28 or functionally equivalents thereof.

| Region | | Sequence | Sequence ID |
|---|---|---|---|
| First Antigen binding region | VH FR1 | EVQLVESGGGLVQPGGSLRLSCAASG FNIK | SEQ ID NO: 13 |
| | VH FR2 | WVRQAPGKGLEWVA | SEQ ID NO: 14 |
| | VH FR3 | RFTISADTSKNTAYLQMNSLRAEDTA VYYCSR | SEQ ID NO: 15 |
| | VH FR4 | WGQGTLVTVSS | SEQ ID NO: 16 |
| | VL FR1 | DIQMTQSPSSLSASVGDRVTITC | SEQ ID NO: 17 |
| | VL FR2 | VAWYQQKPGKAPKLLIY | SEQ ID NO: 18 |
| | VL FR3 | GVPSRFSGSRSGTDFTLTISSLQPED FATYYC | SEQ ID NO: 19 |
| | VL FR4 | FGQGTKVEIKR | SEQ ID NO: 20 |
| Second Antigen binding regiong | VH FR1 | DIKLQQSGAELARPGASVKMSCKTSG YTFT | SEQ ID NO: 21 |
| | VH FR2 | WVKQRPGQGLEWIG | SEQ ID NO: 22 |
| | VH FR3 | KATLTTDKSSSTAYMQLSSLTSEDSA VYYCAR | SEQ ID NO: 23 |
| | VH FR4 | WGQGTTLTVSSVE | SEQ ID NO: 24 |
| | VL FR1 | DIQLTQSPAIMSASPGEKVTMTC | SEQ ID NO: 25 |
| | VL FR2 | WYQQKSGTSPKRWIY | SEQ ID NO: 26 |
| | VL FR3 | GVPYRFSGSGSGTSYSLTISSMEAED AATYYC | SEQ ID NO: 27 |
| | VL FR4 | FGAGTKLELK | SEQ ID NO: 28 |

In a particular embodiment of bispecific antibody expressed by the immune cell of the invention:
- the VH region of the ScFv of the first antigen binding region comprises sequences having at least 74%, at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity with the sequence of SEQ ID NO:29,
- the VL region of the ScFv of the first antigen binding region comprises sequences having at least 74%, at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity with the sequence of SEQ ID NO:30,
- the VH region of the ScFv of the second antigen binding region comprises sequences having at least 74%, at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity with the sequence of SEQ ID NO:31 and/or,
- the VL region of the ScFv of the second antigen binding region comprises sequences having at least 74%, at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity with the sequence of SEQ ID NO:32.

SEQ ID NO:29 
EVQLVESGGGLVQPGGSLRLSCAASGFNIKDTYIHWVRQAPGKGLEWVARIYPTNGYTRYADSVKGRFTI
SADTSKNTAYLQMNSLRAEDTAVYYCSRWGGDGFYAMDVWGQGTLVTVSS
SEQ ID NO: 30 
DIQMTQSPSSLSASVGDRVTITCRASQDVNTAVAWYQQKPGKAPKLLIYSASFLYSGVPSRFSGSRSGTD
FTLTISSLQPEDFATYYCQQHYTTPPTFGQGTKVEIKR
SEQ ID NO: 31 
DIKLQQSGAELARPGASVKMSCKTSGYTFTRYTMHWVKQRPGQGLEWIGYINPSRGYTNYNQKFKDKATL
TTDKSSSTAYMQLSSLTSEDSAVYYCARYYDDHYCLDYWGQGTTLTVSSVE
SEQ ID NO: 32

DIQLTQSPAIMSASPGEKVTMTCRASSSVSYMNWYQQKSGTSPKRWIYDTSKVASGVPYRFSGSGSGTSY
SLTISSMEAEDAATYYCQQWSSNPLTFGAGTKLELK

In a particular embodiment of bispecific antibody expressed by the immune cell of the invention:
- the VH region of the ScFv of the first antigen binding region comprises the sequence of SEQ ID NO:29,
- the VL region of the ScFv of the first antigen binding region comprises the sequence of SEQ ID NO:30,
- the VH region of the ScFv of the second antigen binding region comprises the sequence of SEQ ID NO:31 and/or,
- the VL region of the ScFv of the second antigen binding region comprises the sequence of SEQ ID NO:32.

In a particular embodiment of bispecific antibody expressed by the immune cell of the invention:
- the VH region of the ScFv of the first antigen binding region consists or essentially consists of the sequence of SEQ ID NO:29,
- the VL region of the ScFv of the first antigen binding region consists or essentially consists of the sequence of SEQ ID NO:30,
- the VH region of the ScFv of the second antigen binding region consists or essentially consists of the sequence of SEQ ID NO:31 and/or,
- the VL region of the ScFv of the second antigen binding region consists or essentially consists of the sequence of SEQ ID NO:32.

It will be clear for the expert in the field that the organization of the ScFv domains of the first and second antigen binding regions can vary without affecting its function. Therefore, in particular embodiments, the bispecific antibody expressed by the immune cell of the invention has the following arrangement of regions:
- VL(HER2)-VH(HER2)-VH(CD3)-VL(CD3);
- VL(HER2)-VH(HER2)-VL(CD3)-VH(CD3);
- VH(HER2)-VL(HER2)-VH(CD3)-VL(CD3);
- VH(HER2)-VL(HER2)-VL(CD3)-VH(CD3);
- VL(CD3)-VH(CD3)-VH(HER2)-VL(HER2);
- VL(CD3)-VH(CD3)-VL(HER2)-VH(HER2);
- VH(CD3)-VL(CD3)-VH(HER2)-VL(HER2); or
- VH(CD3)-VL(CD3)-VL(HER2)-VH(HER2).
Wherein VH(HER2) is the VH region of the ScFv of the anti-HER2 antibody, VL(HER2) is the VL region of the ScFv of the anti-HER2 antibody, VH(CD3) is the VH region of the ScFv of the anti-CD3 antibody and VL(CD3) is the VL region of the ScFv of the anti-CD3 antibody

In particular embodiment of bispecific antibody expressed by the immune cell of the invention the VH and VL region of the ScFv of the first antigen binding region, the VH and VL region of the ScFv of the second antigen binding region and/or the ScFv of the first and second antigen binding region are connected by a peptide linker.

In another particular embodiment of bispecific antibody expressed by the immune cell of the invention the linker is selected from SEQ ID NO: 33, SEQ ID NO: 34 or SEQ ID NO: 35.

| | |
|---|---|
| SEQ ID NO: 33 | GGGGSGGGGSGGGGS |
| SEQ ID NO: 34 | GGGGS |
| SEQ ID NO: 35 | GGSGGSGGSGGSGGVD |

In one more particular embodiment of bispecific antibody expressed by the immune cell of the invention
- the ScFv of the first antigen binding region comprises sequences having at least 74%, at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity with the sequence of SEQ ID NO:36,
- the ScFv of the second antigen binding region comprises sequences having at least 74%, at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% sequence identity with the sequence of SEQ ID NO:37.

SEQ ID NO: 36 
DIQMTQSPSSLSASVGDRVTITCRASQDVNTAVAWYQQKPGKAPKLLIYSASFLYSGVPSRFSGSRSGTD
FTLTISSLQPEDFATYYCQQHYTTPPTFGQGTKVEIKRGGGGSGGGGSGGGGSEVQLVESGGGLVQPGGS
LRLSCAASGFNIKDTYIHWVRQAPGKGLEWVARIYPTNGYTRYADSVKGRFTISADTSKNTAYLQMNSLR
AEDTAVYYCSRWGGDGFYAMDVWGQGTLVTVSS
SEQ ID NO: 37

DIKLQQSGAELARPGASVKMSCKTSGYTFTRYTMHWVKQRPGQGLEWIGYINPSRGYTNYNQKFKDKATL
TTDKSSSTAYMQLSSLTSEDSAVYYCARYYDDHYCLDYWGQGTTLTVSSVEGGSGGSGGSGGSGGVDDIQ
LTQSPAIMSASPGEKVTMTCRASSSVSYMNWYQQKSGTSPKRWIYDTSKVASGVPYRFSGSGSGTSYSLT
ISSMEAEDAATYYCQQWSSNPLTFGAGTKLELK

In another particular embodiment of bispecific antibody expressed by the immune cell of the invention
- the ScFv of the first antigen binding region comprises the sequence of SEQ ID NO:36,
- the ScFv of the second antigen binding region comprises the sequence of SEQ ID NO:37.

In one more particular embodiment of bispecific antibody expressed by the immune cell of the invention
- the ScFv of the first antigen binding region consists or essentially consists of the sequence of SEQ ID NO:36,
- the ScFv of the second antigen binding region consists or essentially consists of the sequence of SEQ ID NO:37.

In yet another particular embodiment the bispecific antibody expressed by the immune cell of the invention has sequence of SEQ ID NO: 38.
SEQ ID NO: 38

DIQMTQSPSSLSASVGDRVTITCRASQDVNTAVAWYQQKPGKAPKLLIYSASFLYSGVPSRFSGSRSGTD
FTLTISSLQPEDFATYYCQQHYTTPPTFGQGTKVEIKRGGGGSGGGGSGGGGSEVQLVESGGGLVQPGGS
LRLSCAASGFNIKDTYIHWVRQAPGKGLEWVARIYPTNGYTRYADSVKGRFTISADTSKNTAYLQMNSLR
AEDTAVYYCSRWGGDGFYAMDVWGQGTLVTVSSGGGGSDIKLQQSGAELARPGASVKMSCKTSGYTFTRY
TMHWVKQRPGQGLEWIGYINPSRGYTNYNQKFKDKATLTTDKSSSTAYMQLSSLTSEDSAVYYCARYYDD
HYCLDYWGQGTTLTVSSVEGGSGGSGGSGGSGGVDDIQLTQSPAIMSASPGEKVTMTCRASSSVSYMNWY
QQKSGTSPKRWIYDTSKVASGVPYRFSGSGSGTSYSLTISSMEAEDAATYYCQQWSSNPLTFGAGTKLEL
K

As used herein, the term "binding" in the context of the binding of an antibody/ScFv/FV/CDR to a predetermined antigen or epitope typically is a binding with an affinity corresponding to a KD of about 10⁻⁷ M or less, such as about 10⁻⁸ M or less, such as about 10⁻⁹ M or less, about 10⁻¹⁰ M or less, or about 10⁻¹¹ M or even less when determined by for instance surface plasmon resonance (SPR) technology in a BIAcore 3000 instrument using the antigen as the ligand and the antibody as the analyte, and binds to the predetermined antigen with an affinity corresponding to a K_{D} that is at least ten-fold lower, such as at least 100 fold lower, for instance at least 1,000 fold lower, such as at least 10,000 fold lower, for instance at least 100,000 fold lower than its affinity for binding to a non-specific antigen (e.g., BSA, casein) other than the predetermined antigen or a closely-related antigen. The amount with which the affinity is lower is dependent on the KD of the antibody, so that when the KD of the antibody is very low (that is, the antibody is highly specific), then the amount with which the affinity for the antigen is lower than the affinity for a non-specific antigen may be at least 10,000 fold.

### Uses of the immune cell of the invention

The immune cell of the invention finds uses in medicine, specifically in the treatment of cancer. Therefore, an aspect of the present invention is the immune cell of the invention for use in medicine.

Another aspect of the present invention is the immune cell of the invention for use in a method of preventing or treating cancer.

As used herein, the terms "treat", "treatment", "treatment", or "amelioration". The term refers to therapeutic treatment, the purpose of which is to reverse, reduce, suppress, delay or stop the progression or severity of the condition associated with the disease or disorder. The term "treatment" includes reducing or alleviating at least one adverse effect or condition of a condition, such as cancer, a disease or disorder. Treatment is usually "effective" when one or more symptoms or clinical markers are reduced. Alternatively, treatment is "effective" if disease progression is delayed or halted. That is, "treatment" includes not only the improvement of symptoms or markers, but also the interruption of at least a condition that indicates the progression or worsening of symptoms that would be expected in the absence of treatment. The beneficial or desirable clinical outcome, whether detectable or not, is a reduction in one or more symptoms, a reduction in the extent of the disease, a stable (ie, not aggravated) condition of the disease, a disease These include, but are not limited to, delayed or slowed progression, amelioration or alleviation of the disease state, and remission (partial or total). The term "treatment" of a disease also includes providing relief from symptoms or side effects of the disease (including symptomatic treatment). In some embodiments, treating cancer includes reducing tumor volume, reducing the number of cancer cells, suppressing cancer metastasis, prolonging life, reducing cancer cell growth, reducing cell survival, or reducing cancerous status It involves amelioration of the various physiological symptoms involved.

In certain embodiments of the present disclosure, immune cells are delivered to an individual in need thereof, such as an individual that has cancer. The cells then enhance the individual's immune system to attack the respective cancer cells. In some cases, the individual is provided with one or more doses of the immune cells. In cases where the individual is provided with two or more doses of the immune cells, the duration between the administrations should be sufficient to allow time for propagation in the individual, and in specific embodiments the duration between doses is 1, 2, 3, 4, 5, 6, 7, or more days.

In certain embodiments, a growth factor that promotes the growth and activation of the immune cells is administered to the subject either concomitantly with the immune cells or subsequently to the immune cells. The immune cell growth factor can be any suitable growth factor that promotes the growth and activation of the immune cells. Examples of suitable immune cell growth factors include interleukin (IL)-2, IL-7, IL-15, and IL-12, which can be used alone or in various combinations, such as IL-2 and IL-7, IL-2 and IL-15, IL-7 and IL-15, IL-2, IL-7 and IL-15, IL-12 and IL-7, IL-12 and IL-15, or IL-12 and IL2.

Therapeutically effective amounts of immune cells can be administered by a number of routes, including parenteral administration, for example, intravenous, intraperitoneal, intramuscular, intrasternal, or intraarticular injection, or infusion.

The immune cell population can be administered in treatment regimens consistent with the disease, for example a single or a few doses over one to several days to ameliorate a disease state or periodic doses over an extended time to inhibit disease progression and prevent disease recurrence. The precise dose to be employed in the formulation will also depend on the route of administration, and the seriousness of the disease or disorder, and should be decided according to the judgment of the practitioner and each patient's circumstances. The therapeutically effective number of immune cells will be dependent on the subject being treated, the severity and type of the affliction, and the manner of administration. In some embodiments, a therapeutically effective number of immune cells can vary from about 5 × I06 cells per kg body weight to about 7.5 × I08 cells per kg body weight, such as about 2× I07 cells to about 5x 108 cells per kg body weight, or about 5 × 107 cells to about 2x 108 cells per kg body weight. The exact number of immune cells is readily determined by one of skill in the art based on the age, weight, sex, and physiological condition of the subject. Effective doses can be extrapolated from dose-response curves derived from in vitro or animal model test systems.

In certain embodiments, the compositions and methods of the present embodiments involve an immune cell population in combination with at least one additional therapy. The additional therapy may be radiation therapy, surgery (e.g. lumpectomy and a mastectomy), chemotherapy, gene therapy, DNA therapy, viral therapy, RNA therapy, immunotherapy, bone marrow transplantation, nanotherapy, monoclonal antibody therapy, or a combination of the foregoing. The additional therapy may be in the form of adjuvant or neoadjuvant therapy.

In some embodiments, the additional therapy is the administration of small molecule enzymatic inhibitor or anti-metastatic agent. In some embodiments, the additional therapy is the administration of side- effect limiting agents (e.g. agents intended to lessen the occurrence and/or severity of side effects of treatment, such as anti-nausea agents, etc.). In some embodiments, the additional therapy is radiation therapy. In some embodiments, the additional therapy is surgery. In some embodiments, the additional therapy is a combination of radiation therapy and surgery. In some embodiments, the additional therapy is gamma irradiation. In some embodiments, the additional therapy is therapy targeting PBK/AKT/mTOR pathway, HSP90 inhibitor, tubulin inhibitor, apoptosis inhibitor, and/or chemopreventive agent. The additional therapy may be one or more of the chemotherapeutic agents known in the art.

Various combinations may be employed. For the example below the pharmaceutical composition of the invention or the immune cell therapy is "A" and an anti-cancer therapy is "B":
A/B/A B/A/B B/B/A A/A/B A/B/B B/A/A A/B/B/B B/A/B/B
B/B/B/A B/B/A/B A/A/B/B A/B/A/B A/B/B/A B/B/A/A
B/A/B/A B/A/A/B A/A/A/B B/A/A/A A/B/A/A A/A/B/A

Administration of any compound or therapy of the present embodiments to a patient will follow general protocols for the administration of such compounds, taking into account the toxicity, if any, of the agents. Therefore, in some embodiments there is a step of monitoring toxicity that is attributable to combination therapy.

A wide variety of chemotherapeutic agents may be used in in combination with the pharmaceutical composition of the invention or immune cell therapy. The term "chemotherapy" refers to the use of drugs to treat cancer. A "chemotherapeutic agent" is used to connote a compound or composition that is administered in the treatment of cancer. These agents or drugs are categorized by their mode of activity within a cell, for example, whether and at what stage they affect the cell cycle. Alternatively, an agent may be characterized based on its ability to directly cross-link DNA, to intercalate into DNA, or to induce chromosomal and mitotic aberrations by affecting nucleic acid synthesis.

Examples of chemotherapeutic agents include alkylating agents, such as thiotepa and cyclosphosphamide; alkyl sulfonates, such as busulfan, improsulfan, and piposulfan; aziridines, such as benzodopa, carboquone, meturedopa, and uredopa; ethylenimines and methylamelamines, including altretamine, triethylenemelamine, trietylenephosphoramide, triethiylenethiophosphoramide, and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards, such as chlorambucil, chlomaphazine, cholophosphamide, estramustine, ifosfamide, mechlorethamine, mechlorethamine oxide hydrochloride, melphalan, novembichin, phenesterine, prednimustine, trofosfamide, and uracil mustard; nitrosureas, such as carmustine, chlorozotocin, fotemustine, lomustine, nimustine, and ranimnustine; antibiotics, such as the enediyne antibiotics (e.g., calicheamicin, especially calicheamicin gammall and calicheamicin omegall); dynemicin, including dynemicin A; bisphosphonates, such as clodronate; an esperamicin; as well as neocarzinostatin chromophore and related chromoprotein enediyne antiobiotic chromophores, aclacinomysins, actinomycin, authrarnycin, azaserine, bleomycins, cactinomycin, carabicin, carminomycin, carzinophilin, chromomycinis, dactinomycin, daunorubicin, detorubicin, 6- diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins, such as mitomycin C, mycophenolic acid, nogalarnycin, olivomycins, peplomycin, potfiromycin, puromycin, quelamycin, rodorubicin, streptonigrin, streptozocin, tubercidin, ubenimex, zinostatin, and zorubicin; anti -metabolites, such as methotrexate and 5-fiuorouracil (5-FU); folic acid analogues, such as denopterin, pteropterin, and trimetrexate; purine analogs, such as fludarabine, 6-mercaptopurine, thiamiprine, and thioguanine; pyrimidine analogs, such as ancitabine, azacitidine, 6-azauridine, carmofur, cytarabine, dideoxyuridine, doxifluridine, enocitabine, and floxuridine; androgens, such as calusterone, dromostanolone propionate, epitiostanol, mepitiostane, and testolactone; anti-adrenals, such as mitotane and trilostane; folic acid replenisher, such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids, such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSKpolysaccharide complex; razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; taxoids, e.g., paclitaxel and docetaxel gemcitabine; 6-thioguanine; mercaptopurine; platinum coordination complexes, such as cisplatin, oxaliplatin, and carboplatin; vinblastine; platinum; etoposide (VP- 16); ifosfamide; mitoxantrone; vincristine; vinorelbine; novantrone; teniposide; edatrexate; daunomycin; aminopterin; xeloda; ibandronate; irinotecan (e.g., CPT-11); topoisomerase inhibitor RFS 2000; difluorometlhylornithine (DMFO); retinoids, such as retinoic acid; capecitabine; carboplatin, procarbazine,plicomycin, gemcitabien, navelbine, farnesyl-protein tansferase inhibitors, transplatinum, and pharmaceutically acceptable salts, acids, or derivatives of any of the above.

Also included in the definition of "chemotherapeutic agent" are: (i) anti-hormonal agents that act to regulate or inhibit hormone action on tumors such as anti-estrogens and selective estrogen receptor modulators (SERMs), including, for example, tamoxifen (including NOLVADEX^{®}; tamoxifen citrate), raloxifene, droloxifene, 4-hydroxytamoxifen, trioxifene, keoxifene, LY 1 17018, onapristone, and FARESTON^{®} (toremifine citrate); (ii) aromatase inhibitors that inhibit the enzyme aromatase, which regulates estrogen production in the adrenal glands, such as, for example, 4(5)-imidazoles, aminoglutethimide, MEGASE^{®} (megestrol acetate), AROMASIN^{®} (exemestane; Pfizer), formestanie, fadrozole, RI VISor^{®} (vorozole), FEMARA^{®} (letrozole; Novartis), and ARIMIDEX^{®} (anastrozole; AstraZeneca); (iii) anti-androgens such as flutamide, nilutamide, bicalutamide, leuprolide, and goserelin; as well as troxacitabine (a 1 ,3-dioxolane nucleoside cytosine analog); (iv) protein kinase inhibitors such as ME inhibitors (WO 2007/044515); (v) lipid kinase inhibitors; (vi) antisense oligonucleotides, particularly those which inhibit expression of genes in signaling pathways implicated in aberrant cell proliferation, for example, PKC-alpha, Raf and H-Ras, such as oblimersen (GENASENSE^{®}, Genta Inc.); (vii) ribozymes such as VEGF expression inhibitors (e.g., ANGIOZYME^{®}) and HER2 expression inhibitors; (viii) vaccines such as gene therapy vaccines, for example, ALLOVECTIN^{®}, LEUVECTIN^{®}, and VAX1 D^{®}; PROLEUKIN^{®} rIL-2; topoisomerase 1 inhibitors such as LURTOTECAN^{®}; ABARELIX^{®} rmRH; (ix) anti-angiogenic agents such as bevacizumab (AVASTIN^{®}, Genentech); and pharmaceutically acceptable salts, acids and derivatives of any of the above.

Protein kinase inhibitors include tyrosine kinase inhibitors which inhibit to some extent tyrosine kinase activity of a tyrosine kinase such as an ErbB receptor. Examples of tyrosine kinase inhibitors include EGFR- 10 targeted drugs such as: (i) antibodies which bind to EGFR, including MAb 579 (ATCC CRL HB 8506), MAb 455 (ATCC CRL HB8507), MAb 225 (ATCC CRL 8508), MAb 528 (ATCC CRL 8509) (see, US 4943533, Mendelsohn et al.) and variants thereof, such as chimerized 225 (C225 or Cetuximab; ERBITUX^{®}, Imclone) and reshaped human 225 (H225) (WO 96/40210, Imclone Systems Inc.); antibodies that bind type Π mutant EGFR (US 5212290); humanized and chimeric antibodies that bind EGFR (US 5891996); and human antibodies that bind EGFR, such as ABX-EGF (WO 98/50433); (ii) anti-EGFR antibody conjugated with a cyotoxic agent (EP 659439A2); and small molecules that bind to EGFR including ZD1839 or Gefitinib QRESSA^{™}; Astra Zeneca), Erlotinib HC1 (CP-358774, TARCEVA^{™}; Genentech/OSI) and AG1478, AG1571 (SU 5271; Sugen), quinazolines such as PD 153035,4-(3-chloroanilino) quinazoline, pyridopyrimidines, pyrirnidopyrirnidines, pyrrolopyrimidines, such as CGP 59326, CGP 60261 and CGP 62706, and pyrazolopyrimidines, 4- 20 (phenylamino)-7H-pyrrolo[2,3-dJ pyrimidines, curcumin (diferuloyl methane, 4,5-bis (4- fluoroanilino)phthaiimide), tyrphostines containing nitrothiophene moieties; PD-0183805 (Warner-Lambert) and antisense molecules (e.g., those that bind to ErbB-encoding nucleic acid).

In yet another embodiment, the ADC cytotoxic or chemotherapeutic agent of the present invention is an anti-tubulin agent. In more specific embodiments, the cytotoxic agent is selected from the group consisting of vinca alkaloids, podophyllotoxins, taxanes, baccatin derivatives, cryptophycin, maytansinoids, combretastatins, and dolastatin. In a more specific embodiment, the cytotoxic agent is vincristine, vinblastine, vindesine, vinorelbine, VP-16, camptothecin, paclitaxel, docetaxel, episilon A, episilon B, nocodazole, colchicine, cortisimide, estramustine, semadotine, discodermolide, maytansine DM-1, auristatin or other dolastatin derivatives, such as auristatin E or auristatin F, AEB, AEVB, AEFP, MMAE (monomethyl auristatin E), MMAF (monomethyl auristatin F), eruterobin or netropsin.

In addition, powerful chemotherapeutic agents such as CC-1065 analogs, calikiamycin, maytansine, dolastatin 10 analogs, lysoxin, and palytoxin can be linked to ADCs using conditionally stable linkers to form potent immunoconjugates.

In a particular embodiment, the chemotherapeutic agent is selected from lapatinib, tucatinib and neratinib.

The term "cancer" or "tumour" or "tumour disease", as used herein, refers to a broad group of diseases involving unregulated cell growth and which are also referred to as malignant neoplasms. The term is usually applied to a disease characterized by uncontrolled cell division (or by an increase of survival or apoptosis resistance) and by the ability of said cells to invade other neighboring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumours are classified as being either benign or malignant: benign tumours are tumours that cannot spread by invasion or metastasis, i.e., they only grow locally; whereas malignant tumours are tumours that are capable of spreading by invasion and metastasis. Biological processes known to be related to cancer include angiogenesis, immune cell infiltration, cell migration and metastasis. Cancers usually share some of the following characteristics: sustaining proliferative signalling, evading growth suppressors, resisting cell death, enabling replicative immortality, inducing angiogenesis, and activating invasion and eventually metastasis. Cancers invade nearby parts of the body and may also spread to more distant parts of the body through the lymphatic system or bloodstream. Cancers are classified by the type of cell that the tumour cells resemble, which is therefore presumed to be the origin of the tumour.

Examples of cancer or tumor include without limitation, breast, heart, lung, small intestine, colon, spleen, kidney, bladder, head, neck, ovarian, prostate, brain, rectum, pancreas, skin, bone, bone marrow, blood, thymus, uterus, testicles, hepatobiliary and liver tumors. In particular, the tumor/cancer can be selected from the group of adenoma, angiosarcoma, astrocytoma, epithelial carcinoma, germinoma, glioblastoma, glioma, hemangioendothelioma, hepatoblastoma, leukaemia, lymphoma, medulloblastoma, melanoma, neuroblastoma, hepatobiliary cancer, osteosarcoma, retinoblastoma, rhabdomyosarcoma, sarcoma, teratoma, acrallentiginous melanoma, actinic keratosis adenocarcinoma, adenoid cystic carcinoma, adenosarcoma, adenosquamous carcinoma, astrocytictumors, bartholin gland carcinoma, basal cell carcinoma, bronchial gland carcinoma, carcinosarcoma, cholangiocarcinoma, cystadenoma, endodermal sinus tumor, endometrial hyperplasia, endometrial stromal sarcoma, endometrioid adenocarcinoma, ependymal sarcoma, Swing's sarcoma, focal nodular hyperplasia, germ cell tumors, glucagonoma, hemangioblastoma, hemangioma, hepatic adenoma, hepatic adenomatosis, hepatocellular carcinoma, insulinoma, intraepithelial neoplasia, interepithelial squamous cell neoplasia, invasive squamous cell carcinoma, large cell carcinoma, leiomyosarcoma, malignant melanoma, malignant mesothelialtumor, medulloepithelioma, mucoepidermoid carcinoma, neuroepithelial adenocarcinoma, nodular melanoma, papillary serous adenocarcinoma, pituitary tumors, plasmacytoma, pseudosarcoma, pulmonary blastoma, renal cell carcinoma, serous carcinoma, small cell carcinoma, soft tissue carcinoma, somatostatin-secreting tumor, squamous carcinoma, squamous cell carcinoma, undifferentiated carcinoma, uveal melanoma, verrucous carcinoma, vipoma, Wilm's tumor.

In a particular embodiment, the cancer is a p95HER2 positive cancer.

The terms "p95HER2" and "HER2" have already been defined within the context of the CARs of the invention and said definition applies equally to the present method of diagnosis.

A "cancer that is p95HER2 positive" refers to a cancer in which at least a portion of the cancer cells contain p95HER2, as determined by immunohistochemistry (IHC), Western blot, VeraTag^{®} assay (Monogram Biosciences), or flow cytometry. In some embodiments, a cancer is determined to be p95HER2 positive by IHC. In some such embodiments, a cancer is determined to be p95HER2 positive using the methods described in Sperinde et al., Clin. Canc. Res., 2010, 16(16): 4226-4235, such as methods using anti-p95 antibody clone D9 in a VeraTag assay. In some embodiments, a cancer is determined to be p95HER2 positive using the methods described in U.S. Pat. No. 8,389,227 B2, such as methods using an antibody produced by a hybridoma cell line deposited with the Deutschland Sammlung von Mikroorganismen and Zellen under accession number DSM ACC2904 or DSM ACC2980. In some embodiments, a cancer is determined to be p95HER2 positive according to the assay manufacturer's or assay laboratory's guidelines. p95HER2 refers to a collection of carboxy-terminal HER2 fragments, which, in some embodiments, may be divided into 95- to 100-kDa fragments and 100- to 115-kDa fragments. See, e.g., Arribas et al., Cancer Res., 2011, 71: 1515-1519. In some embodiments, a cancer that is p95HER2 positive contains 100- to 115-kDa fragments of HER2.

### Nucleic acids and vectors of the invention

The immune cell of the invention can be obtained by the use of nucleic acid constructs and/or compositions which encode the CAR and the bispecifc antibody which is to be expressed by the T-cells of the invention.

As such, another aspect of the present invention relates to a nucleic acid construct, from here onwards the nucleic acid construct of the invention, comprising
(i) a first region encoding a chimeric antigen receptor, said chimeric antigen receptor comprising:
   ∘ an antigen binding domain specific for p95HER2,
   ∘ a transmembrane domain and
   ∘ at least one intracellular signaling domain and/or a costimulatory domain
      and
(ii) a second region encoding a bispecific antibody, said bispecific antibody comprising:
   ∘ a first antigen-binding region which specifically binds to HER2 and
   ∘ a second antigen-biding region which specifically binds CD3.
   or a nucleic acid composition, from here onwards the nucleic acid composition of the invention, comprising
   (i) a first polynucleotide encoding a chimeric antigen receptor, said chimeric antigen receptor comprising:
      ∘ an antigen binding domain specific for p95HER2,
      ∘ a transmembrane domain and
      ∘ at least one intracellular signaling domain and/or a costimulatory domain
         and
   (ii) a second polynucleotide encoding a bispecific antibody, said bispecific antibody comprising:
      ∘ a first antigen-binding region which specifically binds to HER2 and
      ∘ a second antigen-biding region which specifically binds CD3.

In particular embodiment of the nucleic acid construct of the invention or of the nucleic acid composition of the invention the CAR antigen binding domain specific for p95HER2, the CAR transmembrane domain, the at least one CAR intracellular signaling domain and/or a CAR costimulatory domain, the bispecific antibody anti-HER2 antigen-binding region and the bispecific antibody anti-CD3 antigen-biding region are as defined in any of the previous aspects and or embodiments.

The term "nucleic acid" or "polynucleotide" refers to deoxyribonucleic acids (DNA) or ribonucleic acids (RNA) and their polymers in either single or double stranded form. Unless specifically limited, the term encompasses nucleic acids containing known analogs of natural nucleotides that have binding capabilities similar to those of a reference nucleic acid and which are metabolized similarly to naturally occurring nucleotides. Unless otherwise indicated, a specific nucleic acid sequence also implies conservatively modified variants (e.g., substitutions with degenerate codons), alleles, orthologs, SNPs and complementary sequences, as well as sequences indicated in direct form. In particular, substitutions with degenerate codons can be obtained by creating sequences in which the third position of one or more selected (or all) codons is replaced by residues with mixed bases and/or deoxyinosine residues.

The term "nucleic acid construct" as used herein refers to one single nucleotide sequence which comprises all the elements required to encode the chimeric antigen receptor and the bispecific antibody, i.e., the first and second regions of the polynucleotide. The term "nucleic acid composition" as used herein refers to a composition comprising at least two nucleotide sequences or polynucleotides, one encoding for the CAR expressed by the immune cell of the invention and the other encoding for the bispecific antibody expressed by the immune cell of the invention.

In a particular embodiment of the nucleic acid construct of the invention or of the nucleic acid composition of the invention the chimeric antigen receptor encoded by the first region of the nucleic acid construct or by the first polynucleotide of the nucleic acid composition and the bispecific antibody encoded by the second region of the nucleic acid construct or by the second polynucleotide of the nucleic acid composition further comprise a signal sequence.

The term "signal sequence", also known as "leader peptide", is used herein according to its ordinary meaning in the art and refers to a peptide having a length of about 5-30 amino acids. A leader peptide is present at the N-terminus of newly synthesized proteins that form part of the secretory pathway. Proteins of the secretory pathway include, but are not limited to proteins that reside either inside certain organelles (the endoplasmic reticulum, Golgi or endosomes), are secreted from the cell, or are inserted into a cellular membrane. In some embodiments, the leader peptide forms part of the transmembrane domain of a protein.

In some embodiments, the isolated nucleic acid encodes a protein from the N-terminus to the C-terminus: a leader peptide is present at the N-terminus of newly synthesized proteins that form part of the secretory pathway.

In a particular embodiment of the nucleic acid construct of the invention or of the nucleic acid composition of the invention the signal sequence forming part of the chimeric antigen receptor comprises the CD8 signal sequence and/or the signal sequence forming part of the bispecific antibody comprises the IgK signal sequence.

In a particular embodiment of the nucleic acid construct of the invention or of the nucleic acid composition of the invention the signal sequence forming part of the chimeric antigen receptor comprises SEQ ID NO:62 and/or the signal sequence forming part of the bispecific antibody comprises SEQ ID NO:63.

| | |
|---|---|
| SEQ ID NO: 62 | MALPVTALLLPLALLLHAARP |
| SEQ ID NO: 63 | MDFQVQIFSFLLISASVIMSRG |

It will be obvious to the expert in the field that the nucleic acid construct of the invention, which comprises two regions, one encoding for the CAR expressed by the immune cell of the invention and the other encoding for the bispecific antibody expressed by the immune cell of the invention, allows two separate polypeptides to be obtained, i.e., the CAR and the bispecific antibody. Several mechanisms are available to produce two or more polypeptides from the same nucleic acid construct. One such way is the existence in said nucleic acid of different transcription promoting elements, e.g., promoters and enhancers, for each of the different coding regions (see aspects and embodiments further below). Another way is the existence of single promoting elements which originate a unique messenger RNA (mRNA) which encodes for both the CAR and the bispecific antibody. In said case the mRNA can be monocistronic, i.e., both regions are contained in the same open reading frame of the mRNA, or bicistronic wherein the mRNA contains two different opening reading frames, one encoding for the first region which corresponds to the CAR expressed by the immune cell of the invention, and the other encoding for the second region which corresponds to the bispecific antibody expressed by the immune cell of the invention.

In a particular embodiment the nucleic acid construct of the invention is a monocistronic construct wherein the chimeric antigen receptor encoded by the first region and the bispecific antibody encoded by the second region are found in the same open-reading frame and are separated by a self-cleaving peptide region.

As used herein, the term "self-cleaving peptide region" means a peptide sequence having a cleavage activity that occurs between two amino acid residues in the peptide sequence itself. Examples of the self-cleaving peptide include a 2A peptide and a 2A-like peptide. For example, in 2A or 2A-like peptides, cleavage occurs between glycine and proline residues on these peptides. This is caused by the "ribosome skipping mechanism", which prevents the formation of normal peptide bonds between glycine residues and proline residues during translation, and does not affect downstream translation. The ribosome skip mechanism is known in the art and is used for the expression of multiple proteins encoded by a single molecule of mRNA. The self-cleaving peptide used in the present invention can be obtained from a viral 2A peptide or a 2A-like peptide having an equivalent function. Examples of 2A self-cleaving peptides include 2A peptides (F2A) derived from foot-and-mouth disease virus (FMDV) (VKQTLNFDLLKLAGDVESNPGP (SEQ ID NO: 87)), 2A peptides (E2A) derived from horse rhinitis A virus (ERAV) (QCTNYALLKLAGDVESNPGP (SEQ ID NO: 88)), 2A peptide (P2A) derived from *Porcine teschovirus* (PTV-1) (ATNFSLLKQAGDVEENPGP (SEQ ID NO: 89)), and 2A peptides (T2A) derived from *Thosea signa* virus (TaV) (EGRGSLLTCGDVEENPGP (SEQ ID NO: 64)). Mutations may be appropriately introduced into the self-cleaving peptide domain as long as its activity is not significantly impaired.

In one more particular embodiment of the nucleic acid construct of the invention the self-cleaving peptide region comprises the sequence of SEQ ID NO: 64.

In another particular embodiment the nucleic acid construct of the invention which is a monocistronic construct wherein the chimeric antigen receptor encoded by the first region and the bispecific antibody encoded by the second region are found in the same open-reading frame and are separated by a cis-acting hydrolase element.

A "cis-acting hydrolase element" or "CHYSEL" refers to a peptide sequence that causes a ribosome to release the growing polypeptide chain that it is being synthesized without dissociation from the mRNA. In this respect, the ribosome continues translating and therefore produces a second polypeptide. Peptides such as the FMDV 2A sequence (GSGSRVTELLYRMKRAETYC PRPLLAIHPTEARHKQKIVAPVKQLLNFDLLKLAGDVESNPGP, SEQ ID NO: 90), Sponge (*Amphimedon queenslandica*) 2A sequence (LLCFLLLLLSGDVELNPGP, SEQ ID NO: 91; or HHFMFLLLLLAGDIELNPGP, SEQ ID NO: 92); acorn worm (*Saccoglossus kowalevskii*) (WFLVLLSFILSGDIEVNPGP, SEQ ID NO: 93) 2A sequence; amphioxus (*Branchiostoma floridae*) (KNCAMYMLLLSGDVETNPGP, SEQ ID NO: 94; or MVISQLMLKLAGDVEENPGP, SEQ ID NO: 95) 2A sequence *Porcine teschovirus-1* (GSGATNFSLLKQAGDVEENPGP, SEQ ID NO: 96) 2A sequence; Thoseaa *signa* virus (GSGEGRGSLLTCGDVEENPGP, SEQ ID NO: 97) 2A sequence; and *Equine rhinitis* A virus (GSGQCTNYALLKLAGDVESNPGP, SEQ ID NO: 98) 2A sequence are CHYSELs of use in this invention. In some embodiments, the 2A sequence is a naturally occurring or synthetic sequence that includes the 2A consensus sequence D-X-E-X-NPGP (SEQ ID NO: 99), in which X is any amino acid residue.

In a particular embodiment the nucleic acid construct of the invention which is a multicistronic construct wherein the chimeric antigen receptor encoded by the first region and the bispecific antibody encoded by the second region are found in different open-reading frames and are separated by an internal ribosome entry site or a cis-acting hydrolase element.

The term "internal ribosome entry site" or "IRES" defines a sequence motif that promotes attachment of ribosomes to that motif on internal mRNA sequences. Consequently, an mRNA containing an IRES sequence motif results in two translational products, one initiating from the 5'-end of the mRNA and the other by an internal translation mechanism mediated by the IRES. A number of IRES have been described and can be used in the nucleic acid construct of this invention. See, e.g., US 8,192,984; WO 2010/119257; and US 2005/0112095.

In addition, the nucleic acid construct of the invention or the nucleic acid composition of the invention may further contain "markers", "labels" or "tags" which may improve or facilitate certain properties of the protein, i.e. protein stability or resistance to degradation, or provide technical advantages during production, i.e., facilitate expression and/or purification. Examples of such tags are biotin, His tag, FLAG tag, Halo tag, MBP tag, HA tag, Myc tag, V5 tag, PA tag, fluorescent protein tag and the like. Said tags can be fused or conjugated to the amino-terminal or the carboxyl-terminal a polypeptide, such as the CAR expressed by the immune cell of the invention or the bispecific antibody expressed by the immune cell of the invention. His tags or "polyhistidines" sequence of histidine amino acids bound by peptide bonds which are fused or conjugated to the amino-terminal end of the protein of the invention, and/or to the carboxyl terminal end of the protein of the invention. In a preferred embodiment the polyhistidine region contains at least 4, at least 5, at least 6, at least 7, at least 8, at least 9, at least 10 histidines.

In a particular embodiment of the nucleic acid construct of the invention or the nucleic acid composition of the invention the chimeric antigen receptor encoded by the first region of the nucleic acid construct or by the first polynucleotide of the nucleic acid composition and/or the bispecific antibody encoded by the second region of the nucleic acid construct or by the second polynucleotide of the nucleic acid composition further contain a tag.

In another particular embodiment of the nucleic acid construct of the invention or the nucleic acid composition of the invention the tag is located at the C-terminus of the bispecific antibody.

In one more particular embodiment of the nucleic acid construct of the invention or the nucleic acid composition according to the invention wherein the tag is a hexahistidine tag.

Another aspect of the present invention relates to a polypeptide encoded by the nucleic acid construct of the invention.

The term "polypeptide" or "peptide" or "protein (if single chain)" are used interchangeably herein and generally refer to amino acid polymers of any length. The polymer can be linear or branched, it can contain modified amino acids, or it can be interrupted by non-amino acids. The term also includes amino acid polymers that have been modified (e.g., disulfide bond formation, glycosylation, lipidation, acetylation, phosphorylation, or any other manipulation, such as conjugation with labeled components). Polypeptides can be isolated from natural sources, can also be produced from eukaryotic or prokaryotic hosts by recombinant technology, or can be artificially synthesized products, as long as they can be expressed.

In a particular embodiment of the polypeptide encoded by the nucleic acid construct of the invention comprises the sequences of SEQ ID NO: 65 or SEQ ID NO: 66.
SEQ ID NO: 65 
MALPVTALLLPLALLLHAARPDIQMTQSPSSLSASVGDRVTITCKASQNVGTAVAWFQQKPGKAPKILIY
SASNRYTGVPSRFSGSGSGTDFTLTISSLQPEDFATYYCQQYSTYPLTFGQGTKLEIKTGSTSGSGKPGS
GEGSEVQLVESGGGLVQPGGSLRLSCAASGFTFSTYGMAWVRQTPDRRLELVATINSNGGKTYHPDSVKG
RFTISRDNSKNTLYLQMNSLRAEDTAVYYCTREGFDYWGQGTLVTVSSTTTPAPRPPTPAPTIASQPLSL
RPEACRPAAGGAVHTRGLDFACDFWVLVVVGGVLACYSLLVTVAFIIFWVRSKRSRLLHSDYMNMTPRRP
GPTRKHYQPYAPPRDFAAYRSRVKFSRSADAPAYQQGQNQLYNELNLGRREEYDVLDKRRGRDPEMGGKP
RRKNPQEGLYNELQKDKMAEAYSEIGMKGERRRGKGHDGLYQGLSTATKDTYDALHMQALPPREGRGSLL
TCGDVEENPGPMDFQVQIFSFLLISASVIMSRGDIQMTQSPSSLSASVGDRVTITCRASQDVNTAVAWYQ
QKPGKAPKLLIYSASFLYSGVPSRFSGSRSGTDFTLTISSLQPEDFATYYCQQHYTTPPTFGQGTKVEIK
RGGGGSGGGGSGGGGSEVQLVESGGGLVQPGGSLRLSCAASGFNIKDTYIHWVRQAPGKGLEWVARIYPT
NGYTRYADSVKGRFTISADTSKNTAYLQMNSLRAEDTAVYYCSRWGGDGFYAMDVWGQGTLVTVSSGGGG
SDIKLQQSGAELARPGASVKMSCKTSGYTFTRYTMHWVKQRPGQGLEWIGYINPSRGYTNYNQKFKDKAT
LTTDKSSSTAYMQLSSLTSEDSAVYYCARYYDDHYCLDYWGQGTTLTVSSVEGGSGGSGGSGGSGGVDDI
QLTQSPAIMSASPGEKVTMTCRASSSVSYMNWYQQKSGTSPKRWIYDTSKVASGVPYRFSGSGSGTSYSL
TISSMEAEDAATYYCQQWSSNPLTFGAGTKLELK
SEQ ID NO: 66

MALPVTALLLPLALLLHAARPDIQMTQSPSSLSASVGDRVTITCKASQNVGTAVAWFQQKPGKAPKILIY
SASNRYTGVPSRFSGSGSGTDFTLTISSLQPEDFATYYCQQYSTYPLTFGQGTKLEIKTGSTSGSGKPGS
GEGSEVQLVESGGGLVQPGGSLRLSCAASGFTFSTYGMAWVRQTPDRRLELVATINSNGGKTYHPDSVKG
RFTISRDNSKNTLYLQMNSLRAEDTAVYYCTREGFDYWGQGTLVTVSSTTTPAPRPPTPAPTIASQPLSL
RPEACRPAAGGAVHTRGLDFACDFWVLVVVGGVLACYSLLVTVAFIIFWVRSKRSRLLHSDYMNMTPRRP
GPTRKHYQPYAPPRDFAAYRSRVKFSRSADAPAYQQGQNQLYNELNLGRREEYDVLDKRRGRDPEMGGKP
RRKNPQEGLYNELQKDKMAEAYSEIGMKGERRRGKGHDGLYQGLSTATKDTYDALHMQALPPREGRGSLL
TCGDVEENPGPMDFQVQIFSFLLISASVIMSRGDIQMTQSPSSLSASVGDRVTITCRASQDVNTAVAWYQ
QKPGKAPKLLIYSASFLYSGVPSRFSGSRSGTDFTLTISSLQPEDFATYYCQQHYTTPPTFGQGTKVEIK
RGGGGSGGGGSGGGGSEVQLVESGGGLVQPGGSLRLSCAASGFNIKDTYIHWVRQAPGKGLEWVARIYPT
NGYTRYADSVKGRFTISADTSKNTAYLQMNSLRAEDTAVYYCSRWGGDGFYAMDVWGQGTLVTVSSGGGG
SDIKLQQSGAELARPGASVKMSCKTSGYTFTRYTMHWVKQRPGQGLEWIGYINPSRGYTNYNQKFKDKAT
LTTDKSSSTAYMQLSSLTSEDSAVYYCARYYDDHYCLDYWGQGTTLTVSSVEGGSGGSGGSGGSGGVDDI
QLTQSPAIMSASPGEKVTMTCRASSSVSYMNWYQQKSGTSPKRWIYDTSKVASGVPYRFSGSGSGTSYSL
TISSMEAEDAATYYCQQWSSNPLTFGAGTKLELKHHHHHH

A further aspect of the present invention relates to a vector containing the nucleic acid construct of the invention, from here onwards the vector of the composition, or a vector composition comprising vectors in which the first and second nucleic acids of the composition are found in different vectors, from here onwards the vector composition of the invention.

As used herein, "vector," "cloning vector," and "expression vector" are vehicles by which the host is transformed and expression of introduced sequences (eg, transcription and translation) may be accomplished. The term "vector" is also meant to include plasmids, phages, viruses and the like.

A nucleotide sequence encoding any of the CARs expressed by the immune cell of the invention and/or the bispecific antibody expressed by the immune cell of the invention can be present in an expression vector and/or a cloning vector. An expression vector can include a selectable marker, an origin of replication, and other features that provide for replication and/or maintenance of the vector. Suitable expression vectors include, e.g., plasmids, viral vectors, and the like.

Large numbers of suitable vectors and promoters are known to those of skill in the art; many are commercially available for generating a subject recombinant constructs. The following vectors are provided by way of example. Bacterial: pBs, phagescript, PsiX174, pBluescript SK, pBs KS, pNH8a, pNH16a, pNH18a, pNH46a (Stratagene, La Jolla, Calif., USA); pTrc99A, pKK223-3, pKK233-3, pDR540, and pRIT5 (Pharmacia, Uppsala, Sweden). Eukaryotic: pWLneo, pSV2cat, pOG44, PXR1, pSG (Stratagene) pSVK3, pBPV, pMSG and pSVL (Pharmacia).

Expression vectors generally have convenient restriction sites located near the promoter sequence to provide for the insertion of nucleic acid sequences encoding heterologous proteins. A selectable marker operative in the expression host may be present. Suitable expression vectors include, but are not limited to, viral vectors, in particular lentiviral (e.g. viral vectors based on vaccinia virus; poliovirus; adenovirus; adeno-associated virus; SV40; herpes simplex virus; human immunodeficiency virus; a retroviral vector (e.g., Murine Leukemia Virus, spleen necrosis virus, and vectors derived from retroviruses such as Rous Sarcoma Virus, Harvey Sarcoma Virus, avian leukosis virus, human immunodeficiency virus, myeloproliferative sarcoma virus, and mammary tumor virus); and the like.

In a particular embodiment of the vector of the invention or of the vector composition of the invention wherein the vector is a lentiviral vector.

The term "promoter" or "regulatory sequence" as used herein refers to a DNA sequence recognized by the transcription machinery of the cell/host required to initiate the specific transcription of a polynucleotide sequence and/or required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue specific manner.

Examples of promoters/regulatory sequences suitable for the present invention are, without limitation human cytomegalovirus promoter (CMV) (Hosseini Rad et al, PLoS One, 2020, 15(7): e0232915), Chinese Hamster Elongation Factor-1a (CHEF-1) (Ebadat, et al, PLoS One, 2017, 12(10):e018596), Human elongation factor-1 alpha (EF-1) (Hosseini Rad et al, PLoS One, 2020, 15(7): e0232915), Human Phosphoglycerate Kinase (hPGK) (Hosseini Rad et al, PLoS One, 2020, 15(7): e0232915), RPBSA (Hosseini Rad et al, PLoS One, 2020, 15(7): e0232915), MND promoter (WO2018085690),murine stem cell virus (MSCV) long terminal repeat (LTR) (Hughes, M. et al., Hum Gene Ther. 2005 Apr; 16(4): 457-472) and the NFAT-minimal IL2 promoter (Hoojberg et al., Blood, 2000 Jul; 96(2): 459-466.

In a particular embodiment of the vector of the invention or of the vector composition of the invention wherein the nucleic acid construct, the first polynucleotide and/or the second polynucleotide are under the operative control of one or more promoters, wherein the promoters can be identical or distinct. In a particular embodiment of the vector of the invention or of the vector composition of the invention wherein the nucleic acid construct, the first polynucleotide is under the operative control of the LTR promoter and the second polynucleotide is under the operative control of the NFAT-minimal IL2 promoter.

In another particular embodiment the vector of the invention or of the vector composition of the invention wherein the nucleic acid construct, the first polynucleotide and/or the second polynucleotide are under operative control of the murine stem cell virus (MSCV) long terminal repeat.

The term "operably linked" or alternatively "transcriptional control" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Operably linked DNA sequences can be contiguous with each other and, e.g., where necessary to join two protein coding regions, are in the same reading frame.

As noted above, in some embodiments, a nucleic acid comprising any of the CARs of the invention will in some embodiments be RNA, e.g., in vitro synthesized RNA. Methods for in vitro synthesis of RNA are known in the art; any known method can be used to synthesize RNA comprising a nucleotide sequence encoding the first and/or the second polypeptide of a heterodimeric, conditionally active CAR of the present disclosure. Methods for introducing RNA into a host cell are known in the art. Introducing RNA comprising a nucleotide sequence encoding the first and/or the second polypeptide of a heterodimeric, conditionally active CAR of the present disclosure into a host cell can be carried out in vitro or ex vivo or in vivo. For example, a host cell (e.g., an NK cell, a cytotoxic T lymphocyte, etc.) can be electroporated in vitro or ex vivo with RNA comprising a nucleotide sequence encoding the first and/or the second polypeptide of a heterodimeric, conditionally active CAR of the present disclosure.

In order to assess the expression of a CAR polypeptide or portions thereof, the expression vector to be introduced into a cell can also contain either a selectable marker gene or a reporter gene or both to facilitate identification and selection of expressing cells from the population of cells sought to be transfected or infected through viral vectors; in other aspects, the selectable marker may be carried on a separate piece of DNA and used in a co- transfection procedure. Both selectable markers and reporter genes may be flanked with appropriate regulatory sequences to enable expression in the host cells. Useful selectable markers include, for example, antibiotic -resistance genes, such as neomycin and the like. Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene. Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter- driven transcription.

### Uses of the nucleic acid construct, nucleic acid composition, vector and/or the vector composition of the invention

Another aspect of the present invention relates to the nucleic acid construct of the invention or nucleic acid composition of the invention or the vector of the invention or vector composition of the invention for use in medicine.

A further aspect of the present invention relates to the nucleic acid construct of the invention or nucleic acid composition of the invention or the vector of the invention or vector composition of the invention for use in a method of preventing or treating cancer.

In a particular embodiment the nucleic acid construct of the invention, the nucleic acid composition of the invention, the vector of the invention or the vector composition of the invention for use in a method of preventing or treating cancer, wherein the cancer is p95HER2 positive.

All the previous definitions and embodiments described are equally valid to the present aspects and their embodiments.

### Methods for obtaining the immune-cells of the invention

The present invention further includes methods that permit to obtain the cells of the invention, which express a CAR and a bispecific antibody. Therefore, one more aspect of the present invention relates to a method, from here onwards the method of the invention, for obtaining a cell expressing
(i) a chimeric antigen receptor comprising:
   ∘ an antigen binding domain specific for p95HER2,
   ∘ a transmembrane domain and
   ∘ at least one intracellular signaling domain and/or a costimulatory domain
      and
(ii) a bispecific antibody comprising:
   ∘ a first antigen-binding region which specifically binds to HER2 and
   ∘ a second antigen-biding region which specifically binds CD3, comprising inserting into the cell a nucleic acid construct of the invention or a nucleic acid composition of the invention or a vector of the invention or vector composition of the invention.

The term "cell" or "engineered cells" means any cell of any organism that is modified, transformed, or manipulated by addition or modification of a gene, a DNA or RNA sequence, or protein or polypeptide. It also refers to the progeny of such cells. Cells or genetically engineered cells of the present invention include isolated immune cells, such as T, NK, or NKT cells that contain the DNA or RNA sequences encoding a CAR or CAR complex specific for p95HER2 and a bispecific antibody specific for HER2 and express said CAR on the cell surface and secrete said bispecific antibody. Isolated cells and engineered cells may be used, for example, for enhancing an NK or NKT cell activity or a T lymphocyte activity, treatment of cancer, and treatment of infectious diseases.

In a particular embodiment of the method of the invention the cell is a mammalian cell.

Suitable mammalian cells include primary cells and immortalized cell lines. Suitable mammalian cell lines include human cell lines, non-human primate cell lines, rodent (e.g., mouse, rat) cell lines, and the like. Suitable mammalian cell lines include, but are not limited to, HeLa cells (e.g., American Type Culture Collection (ATCC) No. CCL-2), CHO cells (e.g., ATCC Nos. CRL9618, CCL61, CRL9096), 293 cells (e.g., ATCC No. CRL-1573), Vero cells, NIH 3T3 cells (e.g., ATCC No. CRL-1658), Huh-7 cells, BHK cells (e.g., ATCC No. CCL10), PC12 cells (ATCC No. CRL1721), COS cells, COS-7 cells (ATCC No. CRL1651), RAT1 cells, mouse L cells (ATCC No. CCLI.3), human embryonic kidney (HEK) cells (ATCC No. CRL1573), HLHepG2 cells, Hut-78, Jurkat, HL-60, NK cell lines (e.g., NKL, NK92, and YTS), and the like.

In some instances, the cell is not an immortalized cell line, but is instead a cell (e.g., a primary cell) obtained from an individual. For example, in some cases, the cell is an immune cell obtained from an individual.

The engineered cells may be obtained from peripheral blood, cord blood, bone marrow, tumour infiltrating lymphocytes, lymph node tissue, or thymus tissue. The host cells may include placental cells, embryonic stem cells, induced pluripotent stem cells, or hematopoietic stem cells. The cells may be obtained from humans, monkeys, chimpanzees, dogs, cats, mice, rats, and transgenic species thereof. The cells may be obtained from established cell lines.

The above cells may be obtained by any known means. The cells may be autologous, syngeneic, allogeneic, or xenogeneic to the recipient of the engineered cells. The term "autologous" refer to any material derived from the same individual to whom it is later to be re-introduced into the individual.

The term "allogeneic" refers to any material derived from a different animal of the same species as the individual to whom the material is introduced. Two or more individuals are said to be allogeneic to one another when the genes at one or more loci are not identical. In some aspects, allogeneic material from individuals of the same species may be sufficiently unlike genetically to interact antigenic ally.

The term "xenogeneic" refers to a graft derived from an animal of a different species.

The term "syngeneic" refers to an extremely close genetic similarity or identity especially with respect to antigens or immunological reactions. Syngeneic systems include for example, models in which organs and cells (e.g. cancer cells and their non-cancerous counterparts) come from the same individual, and/or models in which the organs and cells come from different individual animals that are of the same inbred strain.

In a particular embodiment of the method of the invention the cell is a cell from the immune system.

The immune cell can be obtained from a subject having or diagnosed as having cancer, a plasma cell disorder, or an autoimmune disease or disorder. For example, the immune cell can be obtained from a subject having a cancer, e.g., multiple myeloma, smoldering myeloma, or Waldenstrom's macroglobulenemia. In some embodiments, the immune cell is obtained from a subject resistant to anti-BCMA therapy. immune cells can also be obtained from allogeneic donors, which are non-genetically identical individuals of the same species as the intended recipients of the cells.
immune cells (e.g., human immune cells) that can be used in the invention include autologous cells, obtained from the subject to whom the cells are later to be administered, after ex vivo modification and expansion. For example, the immune cells can be obtained from an individual having or diagnosed as having cancer, a plasma cell disorder, or autoimmune disease or disorder. immune cells can also be obtained from allogeneic donors, which are non-genetically identical individuals of the same species as the intended recipients of the cells. immune cells useful for the invention include T cells and NK cells.

In another particular embodiment of the method of the invention the immune cell is a T cell, a natural killer (NK) cell or a macrophage.

The term "macrophage" refers to a subgroup of phagocytic cells produced by the differentiation of monocytes. Macrophages which are activated by inflammation, immune cytokines or microbial products nonspecifically engulf and kill foreign pathogens within the macrophage by hydrolytic and oxidative attack resulting in degradation of the pathogen. Peptides from degraded proteins are displayed on the macrophage cell surface where they can be recognized by T cells, and they can directly interact with antibodies on the B cell surface, resulting in T and B cell activation and further stimulation of the immune response. Macrophages belong to the class of antigen presenting cells. In one embodiment, the macrophages are splenic macrophages.

In an embodiment, immune cells (e.g., human immune cells) that can be used in the invention include autologous cells, obtained from the subject to whom the cells are later to be administered, after ex vivo modification and expansion. For example, the immune cells can be obtained from an individual having or diagnosed as having cancer. immune cells can also be obtained from allogeneic donors, which are non-genetically identical individuals of the same species as the intended recipients of the cells. immune cells useful for the invention include T, NK and NKT cells.

Methods for obtaining T, NK and NKT cells are known in the art and can be useful for the engineered immune cells described herein. T, NK and NKT cells are typically obtained from peripheral blood that is collected from a subject by, e.g., venipuncture or withdrawal through an implanted port or catheter. Optionally, the blood can be obtained by a process including leukapheresis, in which white cells are obtained from the blood of a subject, while other blood components are returned to the subject. Blood or leukapheresis product (fresh or cryopreserved) is processed to enrich for T, NK or NKT cells using methods known in the art. For example, density gradient centrifugation (using, e.g., Ficoll) and/or counter-flow centrifugal elutriation can be carried out to enrich for mononuclear cells (including T, NK or NKT cells). In one example, for T cells, a T cell stimulation step employing, e.g., CD3/CD28 antibodies coated on magnetic beads or artificial antigen presenting cells (aAPCs) expressing, e.g., cell surface-bound anti-CD3 and anti-CD28 antibody fragments (see below), can further be carried out in order to stimulate T cells and to deplete other cells, e.g., B cells. The T cells of enriched T cell preparations can then be subject to genetic modification.

As an alternative to peripheral blood, tissues including bone marrow, lymph nodes, spleen, and tumors can be used as a source for T cells and NK cells. The T cells and NK cells can be of human, primate, hamster, rabbit, rodent, cow, pig, sheep, horse, goat, dog, or cat origin, but any other mammalian cell may be used. In a certain embodiments of any aspect, the T or NK cell is human.

immune cells such as T, NK or NKT cells can be obtained from a number of sources peripheral blood mononuclear cells, bone marrow, lymph node tissue, cord blood, thymus tissue, tissue from a site of infection, ascites, pleural effusion, spleen tissue, and tumors. Any number of cell lines (e.g. immune cell lines such as T cell lines) available in the art, may also be used.

In an embodiment, immune cells (e.g. T, NK or NKT cells) are obtained from a unit of blood collected from a subject using any suitable techniques known in the art such as Ficoll^{™} separation. In another embodiment, cells from the circulating blood of a subject are obtained by apheresis. The apheresis product typically contains lymphocytes, including T, NK or NKT cells, monocytes, granulocytes, B cells, other nucleated white blood cells, red blood cells, and platelets. It will be appreciated that the cells collected by apheresis may be washed to remove the plasma fraction and to place the cells in an appropriate buffer or media for subsequent processing steps. For example, the cells may be washed with phosphate buffered saline (PBS). Alternatively, the wash solution lacks calcium and may lack magnesium or may lack many if not all divalent cations. Initial activation steps in the absence of calcium can lead to magnified activation. A washing step may be accomplished by methods known to those in the art, such as by using a semi- automated "flow-through" centrifuge (for example, the Cobe 2991 cell processor, the Baxter CytoMate, or the Haemonetics Cell Saver 5) according to the manufacturer's instructions. After washing, the cells may be resuspended in a variety of biocompatible buffers, such as, for example, Ca-free, Mg-free PBS, PlasmaLyte A, or other saline solution with or without buffer. Alternatively, the undesirable components of the apheresis sample may be removed and the cells directly resuspended in culture media.

In an embodiment, immune cells are isolated from peripheral blood lymphocytes by lysing the red blood cells and depleting the monocytes, for example, by centrifugation through a PERCOLLTM gradient or by counter-flow centrifugal elutriation. Specific subpopulations of immune cells, like T cells, such as CD3+, CD28+, CD4+, CD8+, CD45RA+, and CD45RO+T cells, may be further isolated by positive or negative selection techniques known in the art. For example, T cells may be isolated by incubation with anti-CD3/anti-CD28 (e.g., 3x28)-conjugated beads, such as DYNABEADS^{®} M-450 CD3/CD28 T, for a time period sufficient for positive selection of the desired T cells. Additionally or alternatively, a population of T cells may be enriched by negative selection, for instance by a combination of antibodies directed to surface markers unique to the negatively selected cells. Cell sorting and/or selection via negative magnetic immunoadherence or flow cytometry may be used.

It will be understood that cells derived from subjects that are to be modified to express the CAR and the bispecific antibody expressed by the immune cell of the invention may be stored for a period of time prior to their use (see, for example, therapeutic methods below). For example, the cells may be frozen, optionally after they have been washed, or they may be incubated under suitable conditions for them to remain viable until needed (e.g. on a rotator at 2-10°C or at room temperature). In this way, the cells can be stored until such time as they might be needed. They may be stored in an unmodified state (i.e. wherein they do not express the CAR and/or the bispecific antibody) or in a modified state (i.e. wherein they have been modified to express the CAR and/or the bispecific antibody). Prior to use in the therapeutic applications described further below, the cells may be activated and expanded generally using methods known in the art. For example, T cells may be expanded by contact with a surface having attached thereto an agent that stimulates a CD3/TCR complex associated signal and a ligand that stimulates a costimulatory molecule on the surface of the T cells. In particular, T cell populations may be stimulated as described herein, such as by contact with an anti-CD3 antibody, or antigen-binding fragment thereof, or an anti-CD2 antibody immobilized on a surface, or by contact with a protein kinase C activator (eg bryostatin) in conjunction with a calcium ionophore. For co-stimulation of an accessory molecule on the surface of the T cells, a ligand that binds the accessory molecule is used. For example, a population of T cells can be contacted with an anti-CD3 antibody and an anti-CD28 antibody, under conditions appropriate for stimulating proliferation of the T cells. Examples of an anti-CD28 antibody include 9.3, B-T3, XR-CD28 (Diaclone, Besancon, France) can be used as can other methods commonly known in the art.

T cells that have been exposed to varied stimulation times may exhibit different characteristics. For example, typical blood or apherised peripheral blood mononuclear cell products have a helper T cell population (TH, CD4+) that is greater than the cytotoxic or suppressor T cell population (TC, CD8+). Ex vivo expansion of T cells by stimulating CD3 and CD28 receptors produces a population of T cells that prior to about days 8-9 consists predominately of TH cells, while after about days 8-9, the population of T cells comprises an increasingly greater population of TC cells. Accordingly, depending on the purpose of treatment, infusing a subject with a T cell population comprising predominately of TH cells may be advantageous. Similarly, if an antigen-specific subset of TC cells has been isolated it may be beneficial to expand this subset to a greater degree.

In a particular embodiment, the immune cell is a T cell or an NK cell. In a more particular embodiment the T cell is a CD8+ T cell.

Particularly, the host cells of the invention may be expanded prior to transduction with a polynucleotide or vector of the invention.

It is worth noting that after some cancer treatments, in particular, treatments with drugs that damage the immune system, shortly after treatment during the period of time when patients should normally recover from treatment, the quality of the obtained immune cells may be optimal or improved in relation to their ability to reproduce ex vivo. Also, after ex vivo manipulation using the methods described herein, these cells may be in a preferred condition for enhanced engraftment and in vivo propagation. Thus, in connection with the present invention provides for the production of blood cells, including T cells, dendritic cells or other cells of the hematopoietic line, during this phase of recovery. In addition, in some aspects, mobilization modes (e.g., mobilization using GM-CSF) and the establishment of a specific condition can be used to create a condition in a subject in which repopulation, recirculation, regeneration and / or reproduction of specific cell types is advantageous, especially in time of a certain time window after therapy. Illustrative cell types include T cells, B cells, dendritic cells, and other cells of the immune system.

The engineered cells of the present disclosure may also include a suicide system. Suicide systems provide a mechanism whereby the engineered cell, as described above, may be deactivated or destroyed. Such a feature allows precise therapeutic control of any treatments wherein the engineered cells are used. As used herein, a suicide system provides a mechanism by which the cell having the suicide system can be deactivated or destroyed. Suicide systems are well known in the art.

The expression "inserting into the cell a nucleic acid construct of the invention or a nucleic acid composition of the invention or a vector of the invention or vector composition of the invention" as used herein refers the introduction of nucleic acids or vectors into the cells of interest in a way that said nucleic acids or vectors will produce or express the nucleic acids of interest.

Methods of introducing and expressing genes into a cell are known in the art. In the context of one or more expression vectors, the vectors can be readily introduced into a host cell, e.g., mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. A preferred method for the introduction of a polynucleotide into a host cell is calcium phosphate transfection.

Biological methods for introducing a polynucleotide of interest into a host cell include the use of DNA and RNA vectors. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle in vitro and in vivo is a liposome (e.g., an artificial membrane vesicle). In the case where a non-viral delivery system is utilized, an exemplary delivery vehicle is a liposome. The use of lipid formulations is contemplated for the introduction of the nucleic acids into a host cell (in vitro, ex vivo or in vivo). In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

Lipids suitable for use can be obtained from commercial sources. For example, dimyristyi phosphatidylcholine ("DMPC") can be obtained from Sigma, St. Louis, MO; dicetyl phosphate ("DCP") can be obtained from K & K Laboratories (Plainview, NY); cholesterol ("Choi") can be obtained from Calbiochem-Behring; dimyristyi phosphatidylglycerol ("DMPG") and other lipids may be obtained from Avanti Polar Lipids, Inc. (Birmingham, AL). Stock solutions of lipids in chloroform or chloroform/methanol can be stored at about -20°C. Chloroform is used as the only solvent since it is more readily evaporated than methanol.

"Liposome" is a generic term encompassing a variety of single and multilamellar lipid vehicles formed by the generation of enclosed lipid bilayers or aggregates. Liposomes can be characterized as having vesicular structures with a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self - rearrangement before the formation of closed structures and entrap water and dissolved solutes between the lipid bilayers. However, compositions that have different structures in solution than the normal vesicular structure are also encompassed. For example, the lipids may assume a micellar structure or merely exist as non-uniform aggregates of lipid molecules. Also contemplated are lipofectamine- nucleic acid complexes.

In a particular embodiment the method of the invention further comprises a step of selecting the cells which express the CAR in their surface and which secrete the bispecific antibody.

Regardless of the method used to introduce exogenous polynucleotides into a host cell or otherwise expose a cell to the polynucleotide of the present disclosure, in order to confirm the presence of the recombinant DNA sequence in the host cell, a variety of assays may be performed, which will allow for the selection of those cells expressing the CAR on their surface and secreting the bispecific antibody. Such assays include, for example, "molecular biological" assays well known to those of skill in the art, such as Southern and Northern blotting, RT-PCR and PCR; "biochemical" assays, such as detecting the presence or absence of a particular peptide, e.g., by immunological means (ELISAs and Western blots) or by assays described herein to identify agents falling within the scope of the disclosure. It will be obvious to the expert in the art that in order to select the cells expressing both the CAR and the bispecific antibody, two or more techniques of the exemplified above may be used in order to obtain confirmation of the expression of both polypeptides of interest.

The invention is further characterized by the following aspects:
1. An immune cell which expresses
   (i) a chimeric antigen receptor comprising:
      ∘ an antigen binding domain specific for p95HER2,
      ∘ a transmembrane domain and
      ∘ at least one intracellular signaling domain and/or a costimulatory domain
         and
   (ii) a bispecific antibody comprising:
      ∘ a first antigen-binding region which specifically binds to HER2 and
      ∘ a second antigen-biding region which specifically binds CD3.
2. The immune cell according to aspect 1 wherein the first and second antigen binding regions of the bispecific antibody are ScFv.
3. The immune cell according to aspect 2 wherein:
   - the VH region of the ScFv of the first antigen binding region has CDR1, CDR2 and CDR3 which comprise respectively the sequences of SEQ ID NO:1, SEQ ID NO:2 and SEQ ID NO:3,
   - the VL region of the ScFv of the first antigen binding region has CDR1, CDR2 and CDR3 which comprise respectively the sequences of SEQ ID NO:4, the amino acid sequence SAS and SEQ ID NO:6.
   - the VH region of the ScFv of the second antigen binding region has CDR1, CDR2 and CDR3 which comprise respectively the sequences of SEQ ID NO:7, SEQ ID NO:8 and SEQ ID NO:9, and/or
   - the VL region of the ScFv of the second antigen binding region has CDR1, CDR2 and CDR3 which comprise respectively the sequences of SEQ ID NO:10, the amino acid sequence DTS and SEQ ID NO: 12.
4. The immune cell according to aspect 3 wherein:
   - the VH region of the ScFv of the first antigen binding region has FR1, FR2, FR3 and FR4 which comprise respectively the sequences of SEQ ID NO:13, SEQ ID NO:14, SEQ ID NO:15 and SEQ ID NO:16,
   - the VL region of the ScFv of the first antigen binding region has FR1, FR2, FR3 and FR4 which comprise respectively the sequences of SEQ ID NO:17, SEQ ID NO:18, SEQ ID NO:19 or SEQ ID NO:20,
   - the VH region of the ScFv of the second antigen binding region has FR1, FR2, FR3 and FR4 which comprise respectively the sequences of SEQ ID NO:21, SEQ ID NO:22, SEQ ID NO:23 and SEQ ID NO:24, and/or
   - the VL region of the ScFv of the second antigen binding region has FR1, FR2, FR3 and FR4 which comprise respectively the sequences of SEQ ID NO:25, SEQ ID NO:26, SEQ ID NO:27 or SEQ ID NO:28.
5. The immune cell according to aspect 4 wherein:
   - the VH region of the ScFv of the first antigen binding region comprises the sequence of SEQ ID NO:29,
   - the VL region of the ScFv of the first antigen binding region comprises the sequence of SEQ ID NO:30,
   - the VH region of the ScFv of the second antigen binding region comprises the sequence of SEQ ID NO:31 and/or,
   - the VL region of the ScFv of the second antigen binding region comprises the sequence of SEQ ID NO:32.
6. The immune cell according to any preceding aspect wherein the VH and VL region of the ScFv of the first antigen binding region, the VH and VL region of the ScFv of the second antigen binding region and/or the ScFv of the first and second antigen binding region are connected by a peptide linker.
7. The immune cell according to aspect 6 wherein the linker is selected from SEQ ID NO:33, 34 or 35.
8. The immune cell according to aspect 7 wherein
   - the ScFv of the first antigen binding region comprises the sequence of SEQ ID NO:36,
   - the ScFv of the second antigen binding region comprises the sequence of SEQ ID NO:37.
9. The immune cell according to aspect 8 wherein the bispecific antibody has the sequence of SEQ ID NO:38.
10. The immune cell according to any preceding aspect wherein the antigen-binding domain specific for p95HER2 of the CAR is a ScFv.
11. The immune cell according to aspect 10 wherein the CDR1, CDR2 and CDR3 of the VH region of the CAR ScFv comprise, respectively, the sequences of SEQ ID NO: 39, 40 and 41 and/or the CDR1, CDR2 and CDR3 of the VL region of the CAR ScFv comprise respectively, the sequences of SEQ ID NO: 42, 43, and 44.
12. The immune cell according to aspect 11 wherein the FR1, FR2, FR2 and FR4 of the VH region of the CAR comprise respectively the sequences of SEQ ID NO: 45, 46, 47 and 48 and/or the FR1, FR2, FR3 and FR4 of the VL region of the CAR comprise respectively the sequences of SEQ ID NO: 49, 50, 51 and 52 or functionally equivalent variants thereof.
13. The immune cell according to aspect 12 wherein the VH and VL regions of the CAR ScFV are connected by a linker region.
14. The immune cell according to aspect 13 wherein the linker region comprises SEQ ID NO: 53.
15. The immune cell according to aspect 13 wherein the VL of the CAR ScFv comprises the sequence of SEQ ID NO: 54 and the VH of the CAR ScFv comprises the sequence of SEQ ID NO: 55.
16. The immune cell according to aspect 15 wherein the CAR ScFv comprises the sequence of SEQ ID NO:56.
17. The immune cell according to any preceding aspect wherein transmembrane domain of the CAR is selected from the group consisting of the CD4 transmembrane domain, the CD8 transmembrane domain, the CD28 transmembrane domain, the 4-1BB transmembrane domain, the CTLA4 transmembrane domain, the CD27 transmembrane domain and the CD3 zeta transmembrane domain.
18. The immune cell according to aspect 17 wherein the transmembrane domain comprises the sequence of SEQ ID NO:57.
19. The immune cell according to any preceding aspects wherein the at least one intracellular signaling domain of the CAR comprises a costimulatory domain, a primary signaling domain, or any combination thereof.
20. The immune cell according to aspect 18 wherein the at least one intracellular signaling domain comprises the intracellular domain of a costimulatory molecule selected from OX40, CD70, CD27, CD28, CD5, ICAM-1, LFA-1 (CD11a/CD18), ICOS (CD278), DAP10, DAP 12, and 4-1BB (CD137), or any combination thereof.
21. The immune cell according to aspect 20 wherein the intracellular domain of a costimulatory molecule comprises the sequence of SEQ ID NO:58.
22. The immune cell according to any preceding aspect wherein the at least one intracellular signaling domain of the CAR further comprises the sequence of SEQ ID NO:59.
23. The immune cell according to aspect 22 wherein the at least one intracellular signaling domain is arranged on a N-terminal side relative to the CD3 zeta intracellular domain.
24. The immune cell according to any preceding aspect 22 wherein the CAR further comprises a hinge domain between the antigen binding domain and the transmembrane domain.
25. The immune cell according to aspect 24 wherein the hinge domain comprises the sequence of SEQ ID NO:60 (CD8 hinge domain).
26. The immune cell according to aspect 25 wherein the CAR comprises the sequence of SEQ ID NO:61.
27. The immune cell according to any preceding aspects for use in medicine.
28. The immune cell according to any preceding aspects for use in a method of preventing or treating cancer.
29. The immune cell according for use according to aspect 28, wherein the cancer is p95HER2 positive.
30. A nucleic acid construct comprising
   (i) a first region encoding a chimeric antigen receptor, said chimeric antigen receptor comprising:
      ∘ an antigen binding domain specific for p95HER2,
      ∘ a transmembrane domain and
      ∘ at least one intracellular signaling domain and/or a costimulatory domain
         and
   (ii) a second region encoding a bispecific antibody, said bispecific antibody comprising:
      ∘ a first antigen-binding region which specifically binds to HER2 and
      ∘ a second antigen-biding region which specifically binds CD3.
   or a nucleic acid composition comprising
   (i) a first polynucleotide encoding a chimeric antigen receptor, said chimeric antigen receptor comprising:
      ∘ an antigen binding domain specific for p95HER2,
      ∘ a transmembrane domain and
      ∘ at least one intracellular signaling domain and/or a costimulatory domain
         and
   (ii) a second polynucleotide encoding a bispecific antibody, said bispecific antibody comprising:
      ∘ a first antigen-binding region which specifically binds to HER2 and
      ∘ a second antigen-biding region which specifically binds CD3.
31. The nucleic acid construct or the nucleic acid composition according to aspect 30 wherein the CAR antigen binding domain specific for p95HER2, the CAR transmembrane domain, the at least one CAR intracellular signaling domain and/or a CAR costimulatory domain, anti-HER2 antigen-binding region of the bispecific antibody and anti-CD3 antigen-biding region of the are as defined in any of aspects 1 to 26.
32. The nucleic acid construct or the nucleic acid composition according to aspects 30 or 31 wherein the chimeric antigen receptor encoded by the first region of the nucleic acid construct or by the first polynucleotide of the nucleic acid composition and the bispecific antibody encoded by the second region of the nucleic acid construct or by the second polynucleotide of the nucleic acid composition further comprise a signal sequence.
33. The nucleic acid construct or the nucleic acid composition according to aspect 32 wherein the signal sequence forming part of the chimeric antigen receptor comprises SEQ ID NO:62 and/or wherein signal sequence forming part of the bispecific antibody comprises SEQ ID NO:63.
34. The nucleic acid construct of any of aspects 30 to 33 which is a multicistronic construct wherein the chimeric antigen receptor encoded by the first region and the bispecific antibody encoded by the second region are found in the same open-reading frame and are separated by a self-cleaving peptide region.
35. The nucleic acid construct of aspect 34 wherein the self-cleaving peptide region comprises the sequence of SEQ ID NO:64.
36. The nucleic acid construct or the nucleic acid composition according to any of aspects 31 to 35 wherein the chimeric antigen receptor encoded by the first region of the nucleic acid construct or by the first polynucleotide of the nucleic acid composition and/or the bispecific antibody encoded by the second region of the nucleic acid construct or by the second polynucleotide of the nucleic acid composition further contain a tag.
37. The nucleic acid construct or the nucleic acid composition according to aspect 36 wherein the tag is located at the C-terminus of the bispecific antibody.
38. The nucleic acid construct or the nucleic acid composition according to aspect 37 wherein the tag is a hexahistidine tag.
39. A polypeptide encoded by the nucleic acid construct of any of aspects 31 to 38.
40. The polypeptide of aspect 36 which comprises the sequence of SEQ ID NO:65 or SEQ ID NO:66.
41. A vector containing the nucleic acid construct according to any of aspects 31 to 38 or a vector composition comprising vectors in which the first and second nucleic acids of the composition are found in different vectors.
42. The vector or the vector composition of aspect 41 wherein the nucleic acid construct, the first polynucleotide and/or the second polynucleotide are under operative control of the murine stem cell virus (MSCV) long terminal repeat.
43. The vector or the vector composition of aspects 41 or 42 wherein the vector is a lentiviral vector.
44. The nucleic acid construct or nucleic acid composition according to any of aspects 31 to 38 or the vector or vector composition according to any of aspects 41 to 43 for use in medicine.
45. The nucleic acid construct or nucleic acid composition according to any of aspects 31 to 38 or the vector or vector composition according to any of aspects 41 to 43 for use in a method of preventing or treating cancer.
46. The nucleic acid construct, the nucleic acid composition, the vector or the vector composition for use according to aspect 45, wherein the cancer is p95HER2 positive.
47. A method for obtaining a cell expressing
   (i) a chimeric antigen receptor comprising:
      ∘ an antigen binding domain specific for p95HER2,
      ∘ a transmembrane domain and
      ∘ at least one intracellular signaling domain and/or a costimulatory domain
         and
   (ii) a bispecific antibody comprising:
      ∘ a first antigen-binding region which specifically binds to HER2 and
      ∘ a second antigen-biding region which specifically binds CD3.
   which comprises inserting into the cell a nucleic acid construct or a nucleic acid composition according to any of aspects 31 to 38 or a vector or vector composition according to any of aspects 41 to 43 and selecting those cells which express the CAR on their surface and which secrete the bispecific antibody.
48. The method according to aspect 47 wherein the cell is a cell from the immune system.
49. The method according to aspect 48 wherein the cell from the immune system is a T-cell, a NK cell or a macrophage.

The invention will be described by way of the following examples which are to be considered as merely illustrative and not limitative of the scope of the invention.

### EXAMPLES

### Methodology

### Vector design and retrovirus production

Vector plasmids coding for h1-214 p95HER2 CAR (H1-14 CAR), HER2-CD3 BiTE (HER2.BiTE) or p95HER2.CAR HER2-CD3.BiTe (CAR-BITE) were synthesized and cloned into pMSGV-1 retroviral vector (Genscript, Netherlands). Schemes of the different constructs are shown in Figure 2. Then, retrovirus stocks of the aforementioned constructs and empty (UTD) retrovirus were produced. Briefly, 0.7 ug of envelope plasmid (RD-114) and 1.5 ug of transfer plasmid (H1-14 CAR, HER2.BITE, CAR-BITE, UTD in pMSGV-1) were co-transfected in GP2-293 packaging cell line (#631458, Clontech). After 3 days, cell supernatant containing retrovirus particles was collected and store at -80C for future transductions.

### Transduction and expansion of CAR and BiTE secreting T cells

PBMCs were stimulated with 10 ng/ul of anti-CD3 (OKT3) (#16-0037-85, Thermo-Fisher) and 300 U/ml IL-2 (#703892-4, Novartis) for 48 hours before transduction. Then, retroviral supernatants were thawed and centrifuged in retronectin (#T100A, Takara)-coated 6-well plates for 2 hours at 2000g. Next, 2 x 106 stimulated PBMCs were added on top and centrifuged for 10 minutes at 400g. After 5 days, CAR expression and cytotoxic assays were performed. Untransduced T cells (UTD) were transduced with empty CAR retrovirus.

### CAR expression analysis

0.2 × 10⁶ CAR Ts were washed twice with 1xPBS and re-suspended in 1xPBS, 2.5 mM EDTA, 1% BSA, and 5% horse serum for 20 minutes. Then, cells were stained with 1/20 Biotin anti-IgG (#115-065-072, Jackson ImmunoResearch) for 30 minutes and washed twice with 1xPBS. APC-Streptavidin antibody (#405207, Biolegend) at 1/150 and 1/300 anti-CD3-PE (#300408, Biolegend) were added for 30 minutes. Zombie Aqua (#423101, Biolegend) was used as a viability marker at 1:1000 dilution. CAR expression was measured on FACS Celesta (BD Bioscience) and analyzed with FlowJo software.

### CAR T cytotoxic assay

MCF7 p95HER2/empty cells were co-cultured with CAR or BiTE secreting T cells at the indicated ratios in 96-well flat bottom plates. After 48 hours of incubation, the mixture of cells was deattached and washed with 1xPBS and re-suspended in 1xPBS, 2.5 mM EDTA, 1% BSA, and 5% horse serum for 20 minutes. Then, the cell suspension was stained with anti-EpCAM-AF647 (#324212, Biolegend) at 1:300 dilution as a target cell marker and zombie Aqua (#423101, Biolegend) at 1:1000 dilution as a viability marker. EpCAM positive alive target cells were counted on LSR Fortessa (BD Bioscience) and analysed with FlowJo software.

### In vivo patient-derived tumor (PDTXs) models

Patient-derived tumor xenografts (PDTXs) from human breast tumours used in this work were from biopsies or surgical resections at Vall d'Hebron University Hospital, Barcelona, and were obtained following institutional guidelines. The institutional review boards (IRBs) at Vall d'Hebron Hospital provided approval for this study in accordance with the Declaration of Helsinki. Written informed consent was obtained from all patients who provided samples.

Breast cancer fragments of patient samples were implanted orthotopically in NOD.Cg-Prkdcscid II2rgtm1WjI/SzJ (NSG) (#005557, Charles River Laboratories) mice. Tumour xenografts were measured with callipers 2 times a week, and tumour volume was determined using the formula: (length × width2 ) × (π/6).

Once tumour volume reached 200-300 mm³, animals were intravenously (i.v.) treated with 3 × 10⁶ CAR+ or BITE secreting+ T cells and a second dose was administered 10 days later.

### Generation and characterization of NK cells expressing CAR-BiTE

NK cells (CD56+CD3-) were isolated from PBMCs using the NK Cell Isolation Kit (#130-092-657, Miltenyi). NK cells were expanded and activated using NK MACS Medium with 5% AB serum and human cytokines IL-2 (#703892-4, Novartis, 500 U/mL) and IL-15 (#130-095-765, Miltenyi, 140 U/mL) for 48 hours before transduction. Then, cells were transduced as previously explained for T cells. After 5 days, CAR expression was determined similarly to T cells. Cytotoxic assays were performed in MCF7 p95HER2/empty cells co-cultured with CAR-BiTE NK cells at the indicated ratios in 96-well flat bottom plates. After 24 hours of incubation, the mixture of cells was deattached and washed with 1xPBS and re-suspended in 1xPBS, 2.5 mM EDTA, 1% BSA, and 5% horse serum for 20 minutes. Then, the cell suspension was stained with anti-EpCAM-AF647 (#324212, Biolegend) at 1:300 dilution as a target cell marker and zombie Aqua (#423101, Biolegend) at 1:1000 dilution as a viability marker. EpCAM positive alive target cells were counted on LSR Fortessa (BD Bioscience) and analysed with FlowJo software.

### Results

### Second generation p95HER2 CAR Ts have moderate effect against patient-derived tumor xenografts (PDTX)

The antitumor activity of h1-214 anti-p95HER2 CAR against cell-line derived tumors has been previously described in International patent application published as WO2021239965. Now we have tested the efficacy of the p95HER2 CAR on breast cancer patient-derived tumor xenografts. The p95HER2 CAR had no effect on a HER2-positive, p95HER-negative PDTX (Fig. 1A), underscoring the specificity of the CAR. In contrast, the p95HER2 CAR showed anti-tumor efficacy on the p95HER2-postive PDTX (Fig. 1B). The efficacy of the second generation p95HER2 CAR on the PDTX was overtly lower than that on the cell line expressing p95HER2, indicating that the efficacy in the clinic will be limited.

One of the mechanisms behind this resistance in PDTX is tumor antigen heterogeneity and low levels of expression, seen by immunohistochemical analysis of PDTXs samples. In p95HER2 positive tumours, HER2 expression is more abundant and homogeneous than that of p95HER2, although the second is tumor-specific and thus, a safer target. This suggests that p95HER2 could be a safe target for the specific redirection of T cells to the tumor but, once there, targeting HER2 could significantly increase tumor eradication because of its abundance.

### Generation of a Bispecific T cell engager (BiTE) targeting HER2 and CD3

The first step to generate armored CAR Ts was to design the constructs for the expression and secretion of a CD3-HER2 BiTE by T cells, using available public sequences.

We call "HER2-CD3 BiTE" or simply "HER2 BiTE" to a bispecific T cell engager (BiTE) secreted by transduced T cells, that is formed by two scFvs that recognize and bind two antigens respectively: HER2 on tumor cells and CD3 on T cells. Secreted HER2 BiTE, as a dual engager, redirects T cells through CD3 binding towards tumor cells expressing HER2.

### Generation and in vitro characterization of a p95HER2.CAR HER2.BiTe

Once the two individual components were validated, we included the HER2 BiTe to be co-expressed with the p95HER2 CAR as recently described (Choi et al., 2019, Nature Publishing Group 37, 1049-1058). Simplified schemes of the three constructs are shown in Figure 3A. Despite the bigger size of the CAR-BiTE vector, T cells could be retrovirally transduced, although at lower levels than the other two constructs (Fig. 3B).

In vitro cytotoxicity analyses showed that HER2 BiTE secreting T cells and p95HER2 CAR-BiTE T cells targeted MCF7 cells, showing that the HER2 BiTE can target cells expressing HER2 (Fig. 3C), but no killing was observed when T cells only expresses the anti-p95HER2 H1-14 CAR. Results also show that the p95HER2 CAR-BiTE is more efficient than the p95HER2 CAR and HER-BiTE alone, demonstrating the synergistic effect of dual targeting (Fig.3D).

### Effect of a p95HER2. CAR HER2.BiTe on p95HER2-positive PDTX in vivo

NSG mice were orthotopically implanted with fragments of a breast cancer patient-derived tumor xenograft (PDTX), with heterogeneous p95HER2 expression. When tumors reached approximately 300 mm3, mice were treated with 3 x 106 H1 214 p95HER2 CAR+ T cells (H1-14), p95HER2.CAR HER2.BiTE T cells (CAR-BITE) or UTD T cells. The armored CAR BiTE was clearly superior to the second generation p95HER2 h1-14 CAR, which showed very limited antitutomor efficicacy in contrast to the CAR-BITE mediated complete eradication of the tumors (Fig. 4).

### Generation and in vitro characterization of a p95HER2.CAR HER2.BiTe in NK cells

Our constructs could be also used in other immune cells. As an example of this, the CAR-BiTE vector could be retrovirally transduced efficiently also in NK cells, even at higher levels than in T cells (Fig. 5A).

In vitro cytotoxicity analyses showed that the p95HER2 CAR part of the construct does not target HER2 expressing MCF7 cells (Fig. 5B). Given the purification of NK cells previous to these experiments, no CD3+ cells were present and thus no effect of the BiTE part should be expected. Results also show that the p95HER2 CAR-BiTE is efficient in killing cells expressing p95HER2, demonstrating the specificity of the product (Fig. 5B).

## Claims

1. An immune cell which expresses
(i) a chimeric antigen receptor comprising:
∘ an antigen binding domain specific for p95HER2,
∘ a transmembrane domain and
∘ at least one intracellular signaling domain and/or a costimulatory domain
and
(ii) a bispecific antibody comprising:
∘ a first antigen-binding region which specifically binds to HER2 and
∘ a second antigen-biding region which specifically binds CD3.

2. The immune cell according to claim 1 wherein the first and second antigen binding regions of the bispecific antibody are ScFv.

3. The immune cell according to claim 2 wherein:
- the VH region of the ScFv of the first antigen binding region has CDR1, CDR2 and CDR3 which comprise respectively the sequences of SEQ ID NO:1, SEQ ID NO:2 and SEQ ID NO:3,
- the VL region of the ScFv of the first antigen binding region has CDR1, CDR2 and CDR3 which comprise respectively the sequences of SEQ ID NO:4, SEQ ID NO: 100 and SEQ ID NO:6.
- the VH region of the ScFv of the second antigen binding region has CDR1, CDR2 and CDR3 which comprise respectively the sequences of SEQ ID NO:7, SEQ ID NO:8 and SEQ ID NO:9, and/or
- the VL region of the ScFv of the second antigen binding region has CDR1, CDR2 and CDR3 which comprise respectively the sequences of SEQ ID NO:10, SEQ ID NO: 101 and SEQ ID NO: 12.

4. The immune cell according to claim 3 wherein:
- the VH region of the ScFv of the first antigen binding region has FR1, FR2, FR3 and FR4 which comprise respectively the sequences of SEQ ID NO:13, SEQ ID NO:14, SEQ ID NO:15 and SEQ ID NO:16,
- the VL region of the ScFv of the first antigen binding region has FR1, FR2, FR3 and FR4 which comprise respectively the sequences of SEQ ID NO:17, SEQ ID NO:18, SEQ ID NO:19 or SEQ ID NO:20,
- the VH region of the ScFv of the second antigen binding region has FR1, FR2, FR3 and FR4 which comprise respectively the sequences of SEQ ID NO:21, SEQ ID NO:22, SEQ ID NO:23 and SEQ ID NO:24, and/or
- the VL region of the ScFv of the second antigen binding region has FR1, FR2, FR3 and FR4 which comprise respectively the sequences of SEQ ID NO:25, SEQ ID NO:26, SEQ ID NO:27 or SEQ ID NO:28.

5. The immune cell according to claim 4 wherein:
- the VH region of the ScFv of the first antigen binding region comprises the sequence of SEQ ID NO:29,
- the VL region of the ScFv of the first antigen binding region comprises the sequence of SEQ ID NO:30,
- the VH region of the ScFv of the second antigen binding region comprises the sequence of SEQ ID NO:31 and/or,
- the VL region of the ScFv of the second antigen binding region comprises the sequence of SEQ ID NO:32.

6. The immune cell according to any preceding claim wherein the antigen-binding domain specific for p95HER2 of the CAR is a ScFv.

7. The immune cell according to claim 6 wherein the CDR1, CDR2 and CDR3 of the VH region of the CAR ScFv comprise, respectively, the sequences of SEQ ID NO: 39, 40 and 41 and/or the CDR1, CDR2 and CDR3 of the VL region of the CAR ScFv comprise respectively, the sequences of SEQ ID NO: 42, 43, and 44.

8. The immune cell according to claim 7 wherein the FR1, FR2, FR2 and FR4 of the VH region of the CAR comprise respectively the sequences of SEQ ID NO: 45, 46, 47 and 48 and/or the FR1, FR2, FR3 and FR4 of the VL region of the CAR comprise respectively the sequences of SEQ ID NO: 49, 50, 51 and 52 or functionally equivalent variants thereof.

9. The immune cell according to any preceding claims for use in medicine.

10. The immune cell according to any preceding claims for use in a method of preventing or treating cancer.

11. The immune cell according for use according to claim 10, wherein the cancer is p95HER2 positive.

12. A nucleic acid construct comprising
(i) a first region encoding a chimeric antigen receptor, said chimeric antigen receptor comprising:
∘ an antigen binding domain specific for p95HER2,
∘ a transmembrane domain and
∘ at least one intracellular signaling domain and/or a costimulatory domain
and
(ii) a second region encoding a bispecific antibody, said bispecific antibody comprising:
∘ a first antigen-binding region which specifically binds to HER2 and
∘ a second antigen-biding region which specifically binds CD3.
or a nucleic acid composition comprising
(i) a first polynucleotide encoding a chimeric antigen receptor, said chimeric antigen receptor comprising:
∘ an antigen binding domain specific for p95HER2,
∘ a transmembrane domain and
∘ at least one intracellular signaling domain and/or a costimulatory domain
and
(ii) a second polynucleotide encoding a bispecific antibody, said bispecific antibody comprising:
∘ a first antigen-binding region which specifically binds to HER2 and
∘ a second antigen-biding region which specifically binds CD3.

13. The nucleic acid construct or the nucleic acid composition according to claim 30 wherein the CAR antigen binding domain specific for p95HER2, the CAR transmembrane domain, the at least one CAR intracellular signaling domain and/or a CAR costimulatory domain, the anti-HER2 antigen-binding region of the bispecific antibody and the anti-CD3 antigen-binding region of the bispecific antibody are as defined in any of claims 1 to 26.

14. A polypeptide encoded by the nucleic acid construct of claim 12 or 13.

15. A vector containing the nucleic acid construct according to claim 12 or 13, or a vector composition comprising vectors in which the first and second nucleic acids of the composition are found in different vectors.
